(19) Europäisches Patentamt
European Patent Office
Office européen des brevets

(11) Numéro de publication : 0 338 916 B1

(12) # FASCICULE DE BREVET EUROPEEN

(45) Date de publication du fascicule du brevet : **01.07.92 Bulletin 92/27**

(51) Int. Cl.$^5$ : **A61K 37/14**

(21) Numéro de dépôt : **89401076.8**

(22) Date de dépôt : **18.04.89**

(54) **Conjugués macromoléculaires d'hémoglobine, leur procédé de préparation et leurs applications.**

Le dossier contient des informations techniques présentées postérieurement au dépôt de la demande et ne figurant pas dans leprésent fascicule.

(30) Priorité : **20.04.88 FR 8805240**

(43) Date de publication de la demande : **25.10.89 Bulletin 89/43**

(45) Mention de la délivrance du brevet : **01.07.92 Bulletin 92/27**

(84) Etats contractants désignés : **AT BE CH DE ES FR GB GR IT LI LU NL SE**

(56) Documents cités :
**EP-A- 0 140 640**
**DE-A- 3 501 349**
**FR-A- 2 328 478**
**FR-A- 2 551 660**
**FR-A- 2 600 894**

(73) Titulaire : **PASTEUR MERIEUX Sérums et Vaccins**
**58, avenue Leclerc**
**F-69007 Lyon (FR)**

(72) Inventeur : **Dellacherie, Edith**
**15 rue Jean Ploussard**
**F-54220 Malzeville (FR)**
Inventeur : **Leonard, Michèle**
**471 avenue de la Libération Chaligny**
**F-54230 Neuves-Maisons (FR)**
Inventeur : **Sacco, Daniel**
**Bd Charles V**
**F-54000 Nancy (FR)**
Inventeur : **Vigneron, Claude**
**60 quai Claude le Lorrain**
**F-54000 Nancy (FR)**

(74) Mandataire : **Grosset-Fournier, Chantal Catherine et al**
**SC Ernest Gutmann/Yves Plasseraud 67 boulevard Haussmann**
**F-75008 Paris (FR)**

EP 0 338 916 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux conjugués macromoléculaires, leur procédé de préparation et leurs applications comme transporteurs d'oxygène, notamment dans le cadre des transfusions.

On sait qu'il est possible d'injecter par voie intraveineuse une solution aqueuse d'hémoglobine exempte de stroma et rendue isotonique du sang.

Or l'un des inconvénients que présente l'hémoglobine est qu'elle ne reste pas dans le circuit sanguin mais qu'elle diffuse à l'extérieur du système vasculaire, en raison en particulier de sa petite taille.

Afin de remédier à cet inconvénient, c'est à dire afin d'augmenter la persistance intravasculaire de l'hémoglobine libre dans le cadre de son utilisation comme "substitut" du sang ou de soluté de remplissage transporteur d'oxygène, plusieurs procédés ont été utilisés.

On a, par exemple, fixé l'hémoglobine à des macromolécules hydrosolubles dépourvues de toxicité, non antigéniques et hémocompatibles.

C'est ainsi que de nombreux exemples d'hémoglobines modifiées par la fixation chimique de polymères hydrosolubles montrent que le temps de séjour de l'hémoglobine dans l'organisme peut être largement augmenté.

Parmi les différents polymères utilisés, les plus courants sont les polysaccharides et notamment le dextrane (brevet français n° 2.328.478) l'hydroxyéthylamidon (brevet français n° 2.328.478), l'inuline (demande de brevet européen n° 43.675), et les polyalkylèneglycols et plus particulièrement le polyéthylène glycol

Cependant dans tous les cas, l'hémoglobine ainsi modifiée par fixation directe de ces polymères, possède des propriétés oxyphoriques mal adaptées à une utilisation en transfusion sanguine.

On rappelle, en effet, qu'un substitut sanguin ne peut jouer un rôle équivalent à celui qu'assume l'hémoglobine intraérythrocytaire native que dans la mesure où la combinaison qu'il forme avec l'oxygène est réversible, c'est à dire qu'il est capable de fixer l'oxygène, (l'hémoglobine est alors sous forme oxygénée) mais également capable de relarguer facilement l'oxygène (l'hémoglobine est alors sous forme déoxygénée).

Cette propriété vis à vis de l'oxygène est caractérisée par la courbe (appelée courbe de Barcroft) qui correspond à la variation de la quantité d'oxygène retenue par unité de masse du transporteur en fonction de la pression partielle de l'oxygène contenue dans l'atmosphère à laquelle l'hémoglobine est exposée.

Dans le cas d'une solution aqueuse de l'hémoglobine native (concentration 15 μmoles/litre) cette variation est représentée par le tracé de référence représenté sur la figure 1, représenté à pH 7.2 à 25°C.

L'un des paramètres associé à cette courbe est la pression de demi-saturation ($P_{50}$) qui est la pression partielle de l'oxygène à laquelle il faut soumettre la solution d'hémoglobine pour qu'elle absorbe une masse d'oxygène équivalant à 50 % de la quantité maximum à laquelle elle est capable de se combiner. Or dans le cas de l'hémoglobine modifiée (hémoglobine couplée à des polymères) comme indiqué ci-dessus, la courbe de la figure 1 représentant le pourcentage de l'oxygène combiné à l'hémoglobine sous des pressions partielles d'oxygène déterminées, subit une translation vers la gauche par rapport à la courbe correspondant à l'hémoglobine native.

Ceci signifie que la pression $P_{50}$ de l'hémoglobine modifiée est inférieure à celle de l'hémoglobine native; en d'autres termes l'hémoglobine modifiée présente une affinité trop forte vis à vis de l'oxygène, ce qui a pour inconvénient que l'oxygène ne peut être restituée rapidement aux tissus irrigués.

Cette modification des propriétés oxyphoriques de l'hémoglobine modifiée peut s'expliquer notamment pour les raisons suivantes :

– l'hémoglobine native subit à l'intérieur du globule rouge, l'influence du diphospho-2,3-glycérate (2,3 DPG) qui est l'effecteur naturel intraérythrocytaire, qui s'associe avec les amines du site allostérique de la déoxyhémoglobine, ce qui abaisse l'affinité de l'hémoglobine vis à vis de l'oxygène.

Des améliorations pour remédier à cet inconvénient ont été proposées en effectuant le couplage entre des polymères et de l'hémoglobine, en présence d'effecteurs temporaires (ou ligands) tels que le diphospho-2,3-glycérate ou l'inositol hexaphosphate et en absence totale d'oxygène (brevet français n° 2 551 660).

Ces substances polyphosphatées complexent fortement le site allostérique de la déoxyhémoglobine, stabilisant cette dernière, et protègent de cette façon les sites aminés qui sont essentiels pour le processus de transport de l'oxygène.

Cependant ces effecteurs sont éliminés soit au cours des étapes de purification des conjugués polymères de l'hémoglobine, soit dans le plasma, dans le cas où la purification n'a pas entraîné l'élimination des effecteurs, en raison des conditions dissociatives du plasma.

Pour remédier à cet inconvénient, on a proposé d'avoir recours à des effecteurs permanents, par exemple en fixant du pyridoxal-5′-phosphate (Japan Kokai Tokkyo JP n° 59.104.323).

On a également proposé d'utiliser des ligands avec plusieurs groupes phosphates qui, après fixation sur l'hémoglobine, diminuent sensiblement son affinité pour l'oxygène.

Cependant, l'un des inconvénients présentés par ces composés est que leur obtention met en oeuvre plusieurs opérations sur l'hémoglobine et en tout cas plus de deux, ce qui provoque la formation de produits de dégradation, notamment de méthémoglobine.

On a également proposé, dans la demande de brevet France n° 2 600 894, des conjugués macromoléculaires d'hémoglobine, dont la taille est telle que leur diffusivité extravasculaire est limitée, voire annulée, qui sont protégés contre les conditions dissociatives du plasma, dont les propriétés oxyphoriques ne sont pas altérées, mais améliorées par rapport à celles de l'hémoglobine libre, et dont la préparation limite le nombre d'étapes réactionnelles sur l'hémoglobine.

Or, ces composés sont présentés comme étant obtenus par un procédé en deux étapes, à savoir fixation de sites Z portant des charges anioniques sur le polymère P, suivi de la réaction entre le polymère P sur lequel ont été fixés les sites Z et l'hémoglobine, sous forme déoxygénée, en l'absence d'oxygène.

Ce procédé, toutefois simplifié par rapport aux procédés existant, peut être difficile à mettre en oeuvre à l'échelle industrielle, surtout dans le cas où l'on met en oeuvre des volumes importants de solutions de déoxyhémoglobine, la difficulté étant par ailleurs accrue dans la mesure où il est nécessaire de travailler en l'absence d'oxygène.

Or, la Société Demanderesse a trouvé de façon tout à fait inattendue un nouveau procédé d'une part permettant de préparer certains des conjugués qui font l'objet de la demande France n° 2 600 894 et d'autre part permettant d'étendre l'accès de l'homme de l'art à de nouveaux conjugués qui présentent de bonne propriétés oxyphoriques les rendant aptes à une utilisation in vivo comme substitut du sang.

Il s'est en effet avéré que certains conjugués de couplage de l'hémoglobine pouvaient être obtenus en mettant en oeuvre de l'hémoglobine sous forme oxygénée, et en travaillant dans des conditions n'impliquant pas l'absence d'oxygène.

Or, la possibilité de préparer des conjugués macromoléculaires d'hémoglobine en utilisant de l'hémoglobine sous forme oxygénée est toute à fait surprenante et va à l'encontre tant des données connues sur la conformation de l'hémoglobine que des résultats obtenus à ce jour.

En effet, compte tenu des données connues de la conformation de l'hémoglobine et notamment le fait que sous forme déoxy, certaines des amines de l'hémoglobine participent au verrouillage de l'hémoglobine en établissant des ponts salins définis ci-après, ces amines de l'hémoglobine engagées dans les ponts salins ne peuvent donc pas réagir facilement avec le polymère. Lorsque la déoxyhémoglobine et le polymère sont couplés dans le conjugué macromoléculaire d'hémoglobine obtenu, ces amines peuvent alors continuer à assurer la transition de conformation oxy = déoxy de l'hémoglobine. De plus, la déoxyhémoglobine possède un site (site allostérique $\beta$) tapissé de fonctions amines capables de s'associer énergiquement avec des structures renfermant des groupes anioniques. Les polymères portant des charges anioniques vont donc réagir préférentiellement à ce niveau et s'ils portent des fonctions réactives telles que carboxyle, aldéhyde ou OH, réagir à l'intérieur de ce site pour donner des conjugués où l'hémoglobine déoxy est stabilisée, par contre, quand l'hémoglobine est sous forme oxygénée, les ponts salins n'existent plus, et les amines correspondantes peuvent donc réagir avec le polymère, quel qu'il soit. Le résultat de ce couplage conduit à des conjugués dans lesquels l'hémoglobine se désoxygène difficilement, les amines modifiées par le polymère ne pouvant plus établir les ponts salins stabilisant la forme déoxy de l'hémoglobine. Par ailleurs, le site $\beta$ est très désorganisé et l'effet d'orientation par les groupes anioniques du polymère ne peut plus jouer. On obtient ainsi des conjugués possédant une $P_{50}$ très basse.

Ces hypothèses ont été confirmées par les résultats obtenus avec des polymères comportant

– des sites Z comportant des charges anioniques constituées par des phosphates et/ou des sulfates, et/ou des carboxylates et

– des groupes carboxyliques, aldéhyde ou OH non situés sur les susdits sites Z,

et dans lesquels la liaison ionique est établie entre le phosphate et/ou le sulfate, et/ou le carboxylate des sites Z du polymère et l'hémoglobine et la liaison covalente est établie entre les groupes carboxyliques, aldéhyde ou OH situés sur le polymère, et l'hémoglobine.

En effet, lorsque de tels polymères sont mis à réagir avec l'hémoglobine sous forme oxygénée, la $P_{50}$ du conjugué macromoléculaire d'hémoglobine devient inférieure à celle de l'hémoglobine native.

Des résultats obtenus avec des polymères comportant des groupes carboxyliques, lesquels

– d'une part, sous forme carboxylate, servent de charges anioniques établissant des liaisons ioniques entre le polymère et l'hémoglobine

– et, d'autre part, entrent dans l'établissement des liaisons covalentes entre le polymère et l'hémoglobine mais dans lesquels les groupes carboxyliques qui sont engagés dans les liaisons covalentes ne sont pas situés sur les sites Z qui portent les carboxylates qui établissent les liaisons ioniques, ont prouvé que les conjugués d'hémoglobine préparés à partir d'hémoglobine sous forme oxygénée présentent une $P_{50}$ inférieure à celle de l'hémoglobine libre ; en d'autres termes, de tels conjugués d'hémoglobine n'ont pas de

propriétés oxyphoriques intéressantes (cf. Preparative Biochemistry 14(4), 313-329 (1984)).

Or, la possibilité de préparer des conjugués d'hémoglobine présentant des propriétés oxyphoriques intéressantes à partir d'hémoglobine oxygénée a été obtenue grâce au procédé de l'invention.

On a par ailleurs déjà proposé des conjugués macromoléculaires d'hémoglobine dans lesquels

– la liaison ionique entre le polymère et l'hémoglobine est établie entre des sites Z du polymère portant des charges anioniques et l'hémoglobine et

– la liaison covalente est établie

. soit entre des sites Z du polymère et l'hémoglobine,

. soit entre le polymère et l'hémoglobine, sous réserve de conditions entre la nature de la charge anionique, le nombre de charges anioniques et le nombre de monomères.

Or, on a constaté de façon inattendue qu'il suffisait d'au moins une charge anionique par polymère, sous réserve que la charge anionique et la fonction susceptible de former la liaison covalente entre le polymère et l'hémoglobine soit située sur le même site Z.

L'un des buts de l'invention est de fournir un nouveau procédé permettant d'obtenir facilement des conjugués macromoléculaires physiologiquement compatibles, et susceptibles de fixer l'oxygène réversiblement.

L'un des buts de l'invention est de fournir un nouveau procédé permettant d'obtenir facilement des conjugués macromoléculaires susceptibles de restituer l'oxygène plus facilement que l'hémoglobine libre.

L'un des buts de l'invention est de proposer un nouveau procédé permettant d'obtenir des conjugués macromoléculaires faciles à synthétiser à l'échelle industrielle.

L'un des autres buts de l'invention est de fournir un nouveau procédé permettant d'obtenir des conjugués macromoléculaires d'hémoglobine, en soumettant l'hémoglobine au plus à deux étapes, et généralement à une seule étape.

L'un des autres buts de l'invention est de proposer un nouveau procédé permettant d'obtenir facilement des volumes importants de solutions aqueuses contenant des conjugués macromoléculaires d'hémoglobine susceptibles d'être utilisées comme substitut du sang, notamment dans les interventions nécessitant des transfusions ou la perfusion d'organe.

L'un des autres buts de l'invention est de fournir un nouveau procédé permettant d'obtenir facilement des solutions d'hémoglobine dont les propriétés oxyphoriques, révélées par une augmentation de la $P_{50}$ in vitro par rapport à l'hémoglobine libre, demeurent stables in vivo.

L'un des autres buts de l'invention est de proposer un nouveau procédé permettant d'obtenir facilement des composés macromoléculaires d'hémoglobine présentant à la fois une affinité modérée par rapport à l'oxygène, ainsi qu'un volume hydrodynamique élevé, entraînant au cours d'expériences transfusionnelles, l'augmentation de la persistance intravasculaire de l'hémoglobine par suppression de l'hémoglobinurie.

L'un des autres buts de l'invention est de proposer un nouveau procédé permettant d'obtenir facilement des conjugués macromoléculaires d'hémoglobine qui présentent d'excellentes propriétés oxyphoriques révélées par une augmentation de la $P_{50}$ in vitro par rapport à l'hémoglobine libre, dans lesquels le polymère utilisé, mis en présence d'hémoglobine mais dans des conditions de non couplage avec l'hémoglobine, n'entraîne pas nécessairement d'augmentation de la $P_{50}$ par rapport à l'hémoglobine libre.

L'un des buts de l'invention est de proposer de nouveaux conjugués macromoléculaires présentant d'excellentes propriétés oxyphoriques révélées par une augmentation de la $P_{50}$ in vitro et dont la $P_{50}$ puisse être ajustée corrélativement au nombre de liaisons ioniques établies entre le polymère et l'hémoglobine.

Le procédé de préparation de conjugués macromoléculaires hydrosolubles d'hémoglobine, non biodégradables ou peu biodégradables, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce que :

– on fixe, dans une première étape, des sites Z sur un polymère P, à raison d'au moins un site Z par chaîne de polymère, le polymère P étant hydrosoluble, non toxique, de préférence non antigénique, hémocompatible, de masse moléculaire d'environ 1 000 à environ 500 000, de préférence d'environ 1 000 à environ 100 000, comportant un ou plusieurs groupes polaires, de préférence des groupes hydroxyles, carboxyliques ou amines, et les sites Z contenant d'une part au moins une charge négative portée par des groupes, sulfates et/ou phosphates et/ou carboxylates, et destinée à créer une liaison ionique avec l'hémoglobine, et contenant d'autre part au moins un groupe carboxylique, aldéhyde ou OH, destiné à créer une liaison covalente avec l'hémoglobine,

. soit en utilisant un composé Z-Y dans lequel Y est une fonction active ou activable à l'aide d'un agent d'activation, telle que aldéhyde, carboxylique, amine, hydroxyle ou halogène, ou bien

. soit en effectuant un greffage radicalaire des sites Z sur le polymère P ;

– puis dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris d'environ 5 à environ 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine,

– lorsque la réaction ci-dessus indiquée conduit éventuellement à des fonctions imines, celles-ci peuvent être stabilisées en fonctions amines, par exemple par réduction à l'aide de $NaBH_4$, $NaCNBH_3$, le diméthylaminoborane ou HCOOH.

Par commodité de langage, dans la suite du texte, et sauf indication contraire, l'expression "site Z" correspond au site Z après fixation sur le polymère.

Dans ce qui précède et ce qui suit, on utilise l'adjectif "carboxylate" pour désigner la charge anionique - provenant d'un groupe carboxylique - qui intervient dans la liaison ionique entre le polymère et l'hémoglobine et l'adjectif "carboxylique" pour désigner la fonction - provenant également d'un groupe carboxylique, mais différent du précédent - qui établit la liaison covalente entre le polymère et l'hémoglobine.

L'adjectif "carboxylique" englobe également l'anhydride formé à partir de deux groupes carboxyliques.

De plus la charge anionique provenant d'un carboxylate peut également provenir d'une fonction anhydride.

On a constaté qu'il était possible d'utiliser de l'hémoglobine sous forme oxygénée, dans des conditions n'impliquant pas l'absence d'oxygène. Plus précisément, on a constaté :

– que par exemple il est inutile de déoxygéner en boîte à gants et de mettre sous vide les solutions utilisées pour dissoudre le polymère, lorsque celui-ci a été lyophilisé après qu'on y a fixé les sites Z ;

– qu'il est également inutile après déoxygénation des susdites solutions d'effectuer un balayage à l'azote,

– et qu'il est également inutile de traiter l'hémoglobine par les étapes indiquées ci-dessus, à savoir déoxygénation de l'hémoglobine en boîte à gants, mise sous vide et balayage à l'azote et inutile d'avoir à opérer ultérieurement en l'absence d'oxygène, afin d'effectuer le couplage entre le polymère et l'hémoglobine.

On a constaté également que ce procédé n'est applicable que dans la mesure où le site Z porte simultanément :

– des groupes anioniques, choisis parmi les sulfates, phosphates, carboxylates et

– des groupes carboxyliques, aldéhyde ou OH.

En d'autres termes, ce procédé n'est applicable que si une liaison ionique et une liaison covalente sont établies entre le polymère et l'hémoglobine, par l'intermédiaire d'au moins un même site Z porteur des groupes appropriés.

Le procédé de l'invention ne s'applique donc que dans la mesure où :

– il y a établissement d'une liaison ionique entre un groupe phosphate, sulfate ou carboxylate, situé sur un site Z porté par le polymère, et l'hémoglobine,

– et le susdit site Z comporte également un groupe aldéhyde, carboxylique ou OH qui forme une liaison covalente entre le polymère et l'hémoglobine.

En d'autres termes, le procédé de l'invention ne s'applique pas aux polymères comportant

– d'une part des sites Z, porteurs d'un groupe anionique choisi parmi le sulfate, phosphate, carboxylate

– et d'autre part des groupes carboxyliques, aldéhyde ou OH, non situés sur les susdits sites Z.

En d'autres termes, le site Z avant fixation sur le polymère comporte au moins trois groupes fonctionnels qui sont tels que :

– l'une des fonctions est une fonction permettant d'attacher le groupe Z sur le polymère,

– l'une des fonctions est une fonction sulfate, phosphate ou carboxylate susceptible d'établir une liaison ionique avec une amine du site allostérique de l'hémoglobine,

– l'une des fonctions est une fonction aldéhyde, carboxylique ou OH, susceptible de former une liaison covalente avec un groupe $NH_2$ de l'hémoglobine.

Par agent d'activation utilisé pour activer la fonction Y du composé Z-Y dont il est question ci-dessus, on désigne par exemple ceux choisis dans le groupe constitué par les carbodiimides ou le carbonyldiimidazole.

Lorsque le site Z est fixé par greffage radicalaire sur le polymère est utilisé un initiateur radicalaire tel que par exemple le nitrate ou le sulfate cérique d'ammonium ou tout autre agent capable de créer des radicaux libres sur la chaîne polymère (azobis isobutyronitrile, peroxydes, etc...).

Selon un mode de réalisation avantageux du procédé de l'invention, tous les sites Z qui interviennent dans les liaisons covalentes entre le polymère et l'hémoglobine sont les mêmes que les sites Z qui interviennent dans les liaisons ioniques entre le polymère et l'hémoglobine.

Dans le procédé selon l'invention, si les groupes polaires du polymère sont ionisables et s'ils ne sont pas tous engagés dans des liaisons avec les sites Z, ces groupes polaires sont de préférence bloqués afin d'éviter des interactions ultérieures avec les protéines plasmatiques in vivo.

C'est ainsi lorsque les groupes polaires sont des amines, celles qui ne sont pas engagées dans des liaisons

avec les sites Z peuvent être bloquées par exemple à l'aide d'anhydride acétique.

Lorsque les groupes polaires sont des carboxyliques, ceux-ci peuvent être bloqués par exemple à l'aide d'éthanolamine.

Le procédé de l'invention est avantageusement effectué dans des conditions telles que les groupes polaires du polymère sont tous engagés dans les liaisons avec les sites Z, ce qui a pour conséquence qu'il n'y a plus de blocage à effectuer sur les groupes polaires.

Ces conditions consistent notamment à réguler la quantité de sites Z fixés sur le polymère, en fonction des groupes polaires du polymère.

Lorsque les groupes polaires ne sont pas préexistants sur le polymère mis en oeuvre mais doivent être fixés sur le polymère, on peut également contrôler la quantité de groupes polaires qu'on fixe, de façon à ce que le nombre de sites Z fixés sur le polymère soit celui souhaité, sans qu'il y ait pour autant de groupes polaires (résiduels) à bloquer après fixation des sites Z.

Le contrôle du nombre de sites Z peut être très intéressant dans la mesure où il intervient dans la variation de $P_{50}$, par l'intermédiaire de la densité de la charge négative provenant du site Z.

Le contrôle de la fixation des groupes polaires sur le polymère peut se faire de la façon suivante :

Dans l'exemple du dextrane aminé, le nombre de groupes amines peut être modulé en contrôlant la réaction qui précède et qui consiste à faire réagir de l'épichlorhydrine sur le dextrane en présence de catalyseur.

Ce contrôle peut être fait en faisant varier le temps de réaction ou en faisant varier la quantité de catalyseur.

Le contrôle de la fixation des sites Z sur les groupes polaires du polymère lorsque les groupes polaires sont préexistants ou déjà fixés sur le polymère peut se faire de la façon suivante :

- soit en faisant varier le rapport molaire entre les composés Z-Y définis ci-dessus et les groupes polaires du polymère,
- soit en faisant varier le rapport molaire entre l'agent d'activation défini ci-dessus et les groupes polaires du polymère.

Lorsque les groupes Z sont greffés par voie radicalaire, le contrôle de leur fixation se fait indépendament des groupes polaires, en modulant la quantité d'initiateur radicalaire utilisée au cours de la réaction de greffage.

En ce qui concerne la charge anionique, on a constaté qu'il suffisait d'au moins une charge anionique (que ce soit sulfate, phosphate ou carboxylate), par site Z, et qu'il suffisait d'au moins un tel site Z par polymère.

En d'autres termes, on a constaté que le procédé de l'invention était applicable à la préparation de conjugués comportant au moins un seul groupe anionique, et notamment un seul groupe anionique par polymère (lequel groupe anionique n'intervient pas dans le couplage covalent entre le polymère et l'hémoglobine), pour former entre le polymère et l'hémoglobine une liaison ionique.

On a constaté qu'une condition nécessaire pour que les conjugués macromoléculaires obtenus par le procédé de l'invention soient hydrosolubles, dénués de toxicité, de préférence non antigéniques, et hémocompatibles est que les polymères P, susceptibles d'entrer dans la constitution des susdits conjugués macromoléculaires soient hydrosolubles, dénués de toxicité, de préférence non antigéniques, et hémocompatibles.

On a constaté que la présence sur la chaîne du polymère de sites Z, porteurs de groupes anioniques qui jouent le rôle d'effecteurs permanents, augmente la pression partielle pour laquelle 50 % de l'hémoglobine en solution est oxygénée, sans que cela puisse être imputé à l'existence éventuelle de ligands libres.

Ces effecteurs permanents sont tels qu'ils permettent également à l'hémoglobine de passer réversiblement de la forme désoxygénée à la forme oxygénée, avec une stabilisation plus forte de la conformation de la molécule d'hémoglobine sous forme déoxygénée, ce qui entraîne une diminution de l'affinité de l'hémoglobine vis à vis de l'oxygène.

En d'autres termes, ces effecteurs permettent à l'hémoglobine de transporter réversiblement l'oxygène, et notamment de relarguer facilement l'oxygène dans les tissus qui sont irrigués.

La liaison covalente est telle qu'elle confère au conjugué macromoléculaire obtenu par le procédé de l'invention une stabilité le rendant non biodégradable ou peu biodégradable en milieu plasmatique, pendant le temps durant lequel le conjugué macromoléculaire doit assurer des fonctions oxyphoriques, c'est-à-dire 2 à 3 jours, ce qui supprime la diffusion extrarénale et extravasculaire de l'hémoglobine.

Dans les conjugués macromoléculaires obtenus par le procédé de l'invention, l'hémoglobine est reliée au polymère par au moins une liaison covalente mais au-delà d'un nombre critique de liaisons covalentes entre les polymères et l'hémoglobine, notamment quand il y a phénomène de réticulation intermoléculaire, ceci nuit aux propriétés de l'hémoglobine.

Ce nombre critique de liaisons covalentes correspond au fait que la masse moléculaire moyenne en poids des conjugués macromoléculaires de l'invention ne doit pas être supérieure à environ 1 000 000.

Les polymères qui entrent dans la constitution des conjugués macromoléculaires obtenus par le procédé de l'invention qui sont dégradés dans le milieu plasmatique ont une masse moléculaire moyenne en poids

d'environ 1 000 à 500 000.

Les polymères qui entrent dans la constitution des conjugués macromoléculaires obtenus par le procédé de l'invention et qui ne sont pas dégradés dans l'organisme doivent avoir une masse moléculaire moyenne en poids égale ou inférieure à environ 10 000, car au-delà de cette valeur les polymères passent difficilement la barrière rénale et s'accumulent donc dans l'organisme.

C'est notamment le cas des polyalkylène glycols, de la polyvinylpyrrolidone, du polyméthylacrylate ou de certains polysaccharides, qui n'étant pas biodégradables doivent présenter une masse moléculaires moyenne en poids égale ou inférieure à environ 10 000.

Les polymères qui entrent dans la constitution des conjugués macromoléculaires obtenus par le procédé de l'invention doivent être utilisés dans une gamme de poids moléculaire dans laquelle ils sont de préférence non antigéniques.

C'est ainsi que dans le cas du dextrane, son poids moléculaire doit être inférieur à environ 70 000.

La première étape de préparation des conjugués macromoléculaires de l'invention et qui consiste à fixer des sites Z sur le polymère, peut être effectuée par des méthodes connues.

La première étape de préparation des conjugués macromoléculaires de l'invention peut également être effectuée en utilisant des composés du type Z-Y dans lesquels Y est une fonction aldéhyde, carboxylique, amine, hydroxyle ou halogène. Ces composés Z-Y peuvent être utilisés en ayant recours à des techniques chimiques classiques ; on peut par exemple fixer de l'acide benzène penta- ou héxacarboxylique, de l'acide diphosphoro-2,3 glycérique, du pyridoxal-5′-phosphate sur un polymère préalablement polyaminé.

Dans ce cas, il est impératif d'éliminer toute trace du composé Z-Y, par exemple par dessalage sur une colonne de filtration sur gel. En effet, il faut éviter la présence de composé Z-Y qui n'aurait pas réagi et qui, même en faible concentration avec les conjugués macromoléculaires de l'invention peut fausser les conclusions relatives aux propriétés des conjugués de l'invention.

On peut également avoir recours à toute autre méthode permettant de fixer des chaînes polyanioniques sur un polymère, tel qu'une méthode de greffage radicalaire.

La seconde étape mentionnée ci-dessus est une étape de réaction entre le polymère et l'hémoglobine et peut, si nécessaire, être éventuellement précédée d'une phase d'activation du polymère avant que le polymère ne soit mis à réagir avec l'hémoglobine; mais cette activation peut être pratiquement simultanée avec la réaction entre le polymère et l'hémoglobine.

Au cours de la seconde étape, le polymère réagit avec l'hémoglobine et d'une part les liaisons ioniques entre les sites Z du polymère et l'hémoglobine et d'autre part les liaisons covalentes entre le polymère et l'hémoglobine se forment.

Dans le cas où ce sont des groupes carboxyliques des sites Z qui interviennent dans la liaison covalente avec des groupes $NH_2$ de l'hémoglobine, on peut utiliser pour activer les groupes carboxyliques les réactifs classiquement utilisés dans la synthèse peptidique, tels que les carbodiimides hydrosolubles, notamment le chlorhydrate de N′-éthyl-N-(3-diméthylaminopropyl)carbodiimide (EDCI), la N-hydroxysuccinimide, ou l'éthoxy-2-quinoline-1-carboxylate d'éthyle (EEDQ).

La liaison obtenue est alors une liaison amide.

Dans le cas où ce sont les groupes aldéhyde des sites Z qui interviennent dans la liaison covalente avec des groupes $NH_2$ de l'hémoglobine, on peut par exemple avoir recours à l'amination réductive.

L'amination réductive d'un aldéhyde consiste à former une imine par l'action d'une amine, et à réduire simultanément l'imine en amine à l'aide d'un réducteur tel que $NaBH_4$, $NaCNBH_3$, le diméthylaminoborane ou HCOOH.

La liaison obtenue est alors une liaison amine.

Dans le cas où ce sont des groupes aldéhydes des sites Z qui interviennent dans la liaison covalente avec des groupes $NH_2$ de l'hémoglobine, on peut également opérer dans des conditions telles que la liaison obtenue soit une liaison imine, laquelle peut ensuite être stabilisée en amine par réduction avec un réducteur doux, tel que l'un de ceux mentionnés ci-dessus.

Lorsque les groupes Z, ne comportent ni groupes aldéhydes, ni de groupes carboxyliques, mais qu'ils renferment des groupes hydroxyles dont certains sont sur des carbones adjacents, on peut former des groupes aldéhydes, par exemple par oxydation périodique, notamment à l'aide de $NaIO_4$.

La liaison obtenue entre ces groupes aldéhydes et les amines de l'hémoglobine est alors une liaison imine, laquelle peut être stabilisée en amine par réduction, par exemple avec $NaBH_4$.

Lorsque les sites Z ne comportent ni groupes aldéhydes ni groupes carboxyliques, mais comportent une ou plusieurs fonctions OH, les sites Z peuvent être mis en jeu sur des groupes $NH_2$ de l'hémoglobine à l'aide d'un réactif approprié tel que le carbonyldiimidazole. Dans ce cas, la liaison obtenue est une liaison carbamate

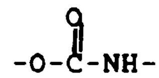

Les conjugués macromoléculaires d'hémoglobine dans lesquels les liaisons covalentes entre le polymère sont des liaisons imines peuvent ne pas être stables dans l'organisme, c'est pourquoi il convient de stabiliser la fonction imine en amine, par exemple par réduction par $NaBH_4$, $NaCNBH_3$, ou le diméthylaminoborane.

Dans le cas où il est nécessaire de stabiliser les liaisons covalentes, l'hémoglobine n'est en tout cas pas soumise à plus de deux étapes de réaction, et dans les autres cas, qu'il y ait activation ou non du polymère P, l'hémoglobine est soumise à une seule étape de réaction, ce qui est particulièrement avantageux car le nombre limité d'étapes de réaction sur l'hémoglobine (au plus égal à deux) est l'une des conditions essentielles pour que, en plus du rendement de la réaction, l'hémoglobine ne soit pas dénaturée.

Au cours de la deuxième étape, il est en effet essentiel que l'hémoglobine ne subisse au cours de la formation du conjugué macromoléculaire de l'invention ni dénaturation importante, ni réduction sensible de la mobilité relative des diverses entités qui la constituent afin de conserver, au moins en partie, ses propriétés oxyphoriques.

La deuxième étape de réaction a lieu en milieu aqueux, tamponné ou non, à un pH d'environ 5 à environ 9, éventuellement en présence d'un activateur comme indiqué ci-dessus, pendant une durée suffisante et de préférence inférieure à celle à partir de laquelle il y a formation importante de methémoglobine (supérieure à environ 5 %), à une température assurant une conservation correcte de l'hémoglobine.

Le milieu aqueux est tamponné par des tampons classiquement utilisés, pour stabiliser le pH à la valeur souhaitée.

La durée de réaction est d'environ 30 mn à environ 20 h, et avantageusement d'environ 1 h à environ 8 h, à une température d'environ 3 à environ 30°C.

La durée de la réaction dépend notamment de la température à laquelle on travaille.

L'hémoglobine utilisée est sous forme oxygénée, et on utilise avantageusement une solution d'hémoglobine à 10 %.

Le rapport des concentrations molaires entre le polymère et l'hémoglobine doit en outre être tel que la plus grande partie des molécules d'hémoglobine soit liée de façon covalente avec le polymère polyanionique.

A titre d'exemple, on peut indiquer qu'en faisant réagir, à une température d'environ 3 à 30°C, en solution dans un milieu aqueux à pH voisin de 6,5, un mélange de polymère dextrane, dont la masse moléculaire moyenne en poids est d'environ 10 000 à environ 40 000, et d'hémoglobine dans lequel le rapport des concentrations molaires entre le dextrane et l'hémoglobine est d'environ 0,5 à environ 5, on obtient des conjugués macromoléculaires de l'invention, dont l'examen chromatographique, par exemple par perméation sur gel montre qu'il n'y a plus d'hémoglobine libre.

Selon un mode de réalisation avantageux, le procédé de l'invention est tel qu'aucune liaison ionique et aucune liaison covalente n'est susceptible de s'établir ailleurs qu'entre les groupes respectifs appropriés des sites Z du polymère et l'hémoglobine.

Un procédé de préparation de conjugués macromoléculaires avantageux de l'invention est caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et l'hémoglobine) soit la suivante :

– lorsque chaque site Z contient un groupe anionique unique constitué d'un sulfate ou d'un phosphate, il y a au moins un tel site Z tous les dix monomères du polymère,

– lorsqu'il s'agit de site Z contenant au moins deux groupes anioniques, constitués de sulfates et/ou de phosphates, il y a au moins un tel site Z par chaîne de polymère,

– lorsque chaque site Z contient des charges anioniques provenant de carboxylates, il faut au moins deux groupes carboxylates - non engagés dans la liaison covalente entre le polymère et l'hémoglobine - sur un même site Z, et au moins un tel site Z tous les cinq monomères,

– lorsqu'il s'agit de site Z contenant au moins trois charges négatives provenant de carboxylates

– non engagés dans la liaison covalente entre le polymère et l'hémoglobine - il y a au moins un tel site Z par chaîne de polymère.

La relation entre le nombre de sites Z et de monomères est statistique dans la mesure où par exemple, lorsque il est indiqué qu'il y a au moins un site Z par chaîne de polymère, cela signifie que certaines chaînes de polymère ne peuvent comporter aucun site Z et certaines autres chaînes peuvent comporter deux sites, mais il est bien évident que, pour être active vis-à-vis de l'hémoglobine, une chaîne de polymère doit contenir

au moins un site Z.

Selon un autre mode de réalisation avantageux, le procédé de l'invention de préparation de conjugués macromoléculaires est caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et le polymère) soit la suivante:

– lorsqu'il s'agit de site Z contenant un groupe anionique unique constitué d'un sulfate ou d'un phosphate, il y a au moins un tel site Z par chaîne de polymère et au plus un site Z tous les onze monomères,

– lorsqu'il s'agit de site Z contenant un groupe anionique unique constitué d'un carboxylate - non engagé dans la liaison entre le site et le polymère et non engagé dans la liaison covalente entre le polymère et l'hémoglobine - il y au moins un tel site Z par chaîne de polymère,

– lorsqu'il s'agit de site Z contenant deux charges anioniques provenant de deux carboxylates - non engagés dans la liaison entre le site et le polymère et non engagés dans la liaison covalente entre le polymère et l'hémoglobine -, il y a au moins un tel site Z par chaîne de polymère et au plus un tel site Z tous les six monomères.

Les conjugués d'hémoglobine obtenus par mise en oeuvre selon cette variante du procédé de l'invention sont nouveaux.

Selon un mode de réalisation avantageux, le procédé selon l'invention est caractérisé en ce que les liaisons covalentes entre le polymère P et l'hémoglobine sont établies entre au moins un groupe carboxylique ou aldéhyde ou OH porté par les sites Z et au moins une amine de l'hémoglobine située dans le site allostérique de l'hémoglobine, notamment l'amine de l'une au moins des deux valines β-terminales de l'hémoglobine.

Selon un mode de réalisation avantageux du procédé de l'invention, les ponts salins entre les groupes $NH_3^+$ et les groupes $COO^-$ internes de l'hémoglobine sont intacts lorsque l'hémoglobine est sous forme déoxygénée.

On désigne par ponts salins, certaines liaisons intra-moléculaires qui se forment entre des ions $NH_3^+$ et des anions $COO^-$, lorsque l'hémoglobine est sous forme désoxygénée.

Les ponts salins dans les conjugués macromoléculaires obtenus par le procédé de l'invention doivent de préférence être intacts, car si les ions qui participent à la formation de ces ponts salins, sont engagés dans d'autres liaisons, le passage de l'hémoglobine de la forme oxygénée à la forme désoxygénée se fait très difficilement et de manière incomplète.

L'expression "ponts salins intacts" signifie qu'au moins 50 % des ponts salins ne sont pas altérés, et qu'avantageusement 80 à 90 %, voire 100 %, des ponts salins ne sont pas altérés.

Les critères permettant de vérifier si les ponts salins sont intacts sont notamment les suivants :

a) Courbe d'affinité vis à vis de l'oxygène :

si la courbe d'affinité vis à vis de l'oxygène (courbe de Barcroft) est déplacée vers la droite par rapport à celle de l'hémoglobine libre, ceci implique que les ponts salins ne sont pas modifiés.

b) Coefficient de Hill (n) :

ce paramètre traduit l'allure sigmoïde de la courbe de Barcroft et reflète le caractère plus ou moins coopératif de la fixation de l'oxygène. La valeur de n permet d'apprécier la permanence du comportement allostérique de l'hémoglobine. Dans le cas de l'hémoglobine native, ce coefficient est compris entre 2,7 et 3,0.

c) Effet Bohr :

il consiste à déterminer le comportement oxyphorique de l'hémoglobine à différents pH, ce qui permet d'évaluer les perturbations consécutives aux différentes manipulations.

Selon un autre mode de réalisation avantageux les conjugués macromoléculaires de l'invention sont tels que les polymères sont choisis parmi les polysaccharides, notamment les hydroxyalkylamidons dont le groupe alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkylène glycols dans lesquels le groupe alkylène comporte de 2 à 5 atomes de carbone, notamment le polyéthylène glycol et le polypropylène glycol.

Les conjugués d'hémoglobine obtenus selon le procédé de l'invention sont tels que les liaisons ioniques entre le polymère et l'hémoglobine sont établies entre les groupes phosphate, sulfate ou carboxylate des sites Z du polymère et les groupes amines de l'hémoglobine.

Selon un mode de réalisation avantageux de l'invention, les conjugués macromoléculaires sont tels que les liaisons ioniques sont établies entre les groupes carboxylates des sites Z du polymère et les groupes $NH_2$ de l'hémoglobine.

Selon un autre mode de réalisation avantageux de l'invention, les sites Z comportent $OSO_3H$ ou $OPO_3H_2$.

Selon un autre mode de réalisation avantageux de l'invention, les sites Z proviennent du pyridoxalsulfate, du disulfate d'épinéphrine, du trisulfate d'épinéphrine, du disulfate de norépinéphrine, du trisulfate de norépinéphrine, du disulfate de phénolphtaleïne.

Selon un autre mode de réalisation avantageux de l'invention, les sites Z proviennent du pyridoxalphosphate, de l'adénosine triphosphate, de la phosphotyrosine, de la phosphosérine, de l'inositolhéxaphosphate et ses dérivés, de l'inositol tri-, tétra-, pentaphosphate et ses dérivés.

Selon un autre mode de réalisation avantageux de l'invention, les sites Z comportent les groupes suivants :

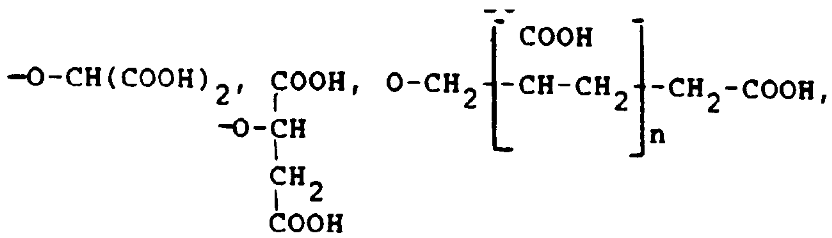

n variant 1 à 4.

Selon un autre mode de réalisation avantageux de l'invention, le site Z provient :

– d'un acide carboxylique contenant au moins un groupe carboxylate sur la chaîne principale non engagé dans la liaison entre le polymère et le site Z et non engagé dans la liaison covalente entre le site Z et l'hémoglobine,

– d'un acide benzènecarboxylique comportant au moins une fonction carboxylique non engagée dans la liaison entre le polymère et le site Z et non engagé dans la liaison covalente entre le site Z et l'hémoglobine,

– d'un groupe diphospho 2,3-glycérate,

– de l'acide citrique,

– du propane-1, 2, 3-tricarboxylate

– ou du butane-tétracarboxylate.

Selon un autre mode de réalisation avantageux de l'invention Z provient de l'hydroxy-2-formyl-5-phosphosérine benzamide de formule :

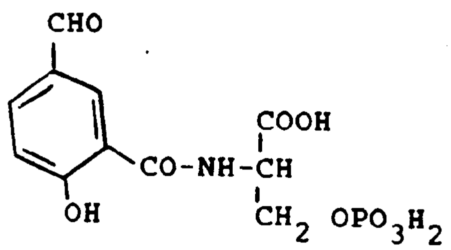

ou du formyl-4-phosphosérine benzamide de formule :

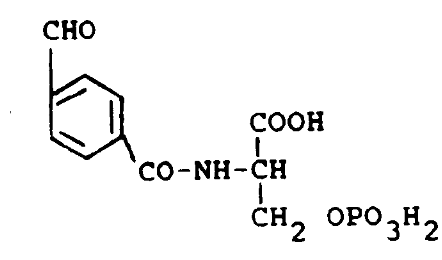

La nature de la liaison qui relie Z au polymère dépend des éléments réactionnels en présence et des conditions de réaction.

Les liaisons qui unissent Z au polymère sont des liaisons éther, ester, amide ou amine.

La liaison éther est obtenue par greffage radicalaire de l'acide acrylique, ou par action d'acide chlorosuccinique, sur les groupes OH des polymères ; la liaison amide est obtenue par réaction entre les groupes carboxylates des groupes Z et les groupes $NH_2$ des polymères; la liaison amine est obtenue par réaction entre

les groupes aldéhydes des groupes Z et les groupes $NH_2$ des polymères, suivie d'une réduction. Cette dernière réaction peut également être effectuée simultanément à la première :amination réductive.

A titre d'exemple, la liaison ester est obtenue

. soit par action des groupes carboxyliques d'un site Z, sous forme anhydride, sur les fonctions OH d'un polymère,

. soit par action d'un groupe carboxylique d'un site Z sur les fonctions OH d'un polymère en présence d'agents de condensation tels que les carbodiimides.

Selon un mode de réalisation avantageux du procédé de préparation, les conjugués de l'invention dans lesquels les sites Z sont reliés au polymère par l'intermédiaire d'une liaison ester peuvent être obtenus de la facon suivante:

– dans une première étape, on fait réagir les sites Z dans lesquels les groupes carboxyliques sont sous forme anhydride, sur un polymère comportant des fonctions OH, dans un milieu dans lequel le polymère est soluble, pour fixer les sites Z sur le polymère;

– dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris d'environ 5 à environ 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine.

Selon un autre mode de réalisation avantageux du procédé de préparation, les conjugués de l'invention dans lesquels les sites Z sont reliés au polymère par l'intermédiaire d'une liaison ester peuvent être obtenus de la façon suivante:

– dans une première étape, on fait réagir les sites Z comportant des groupes carboxyliques, sur un polymère comportant des fonctions OH, en présence d'un agent de condensation tel qu'une carbodiimide, pour fixer les sites Z sur le polymère;

– dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris de 5 à 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine.

Les carbodiimides utilisées dépendent du milieu dans lequel le polymère est soluble. Lorsque le polymère est du polyéthylène glycol, on peut utiliser une carbodiimide soluble dans l'eau ou dans un milieu organique. Lorsque le polymère est du dextrane, étant donné que le polymère est soluble uniquement dans l'eau, la carbodiimide utilisée est avantageusement le chlorhydrate de N'-éthyl-N(3-diméthylaminopropyl) carbodiimide (EDCI).

Comme exemple de polymères comportant des groupes OH on peut citer:

le dextrane, le polyéthylèneglycol.

Par "site Z dans lesquels les groupes carboxyliques sont sous forme anhydride", on entend aussi bien les sites Z comportant deux groupes carboxyliques sous forme anhydride (monoanhydrides) que ceux comportant un nombre pair de groupes carboxyliques dans lesquels chaque paire de groupes carboxyliques est sous forme anhydride (polyanhydrides: par exemple dianhydride ou trianhydride).

Comme exemple de sites Z dans lesquels les groupes carboxyliques sont sous forme anhydride on peut citer, outre les anhydrides d'acide polycarboxyliques, tels que le benzène-1,2,4,5-tétracarboxylique-dianhydride, le benzène-1,2,4-tricarboxylique anhydride, également le cyclobutane-1,2,3,4-tétracarboxylique-dianhydride, la benzophénone-tétracarboxylique dianhydride, l'anhydride de l'acide aconitique.

Le procédé de l'invention met avantageusement en oeuvre, à titre de site Z, le dianhydride d'acide polycarboxylique dans lequel l'un des groupes carboxyliques de l'une des fonctions anhydrides est engagé dans la liaison covalente entre le polymère et l'hémoglobine, et les autres groupes carboxyliques (dont ceux provenant des fonctions anhydrides) servent à la liaison ionique et à la liaison covalente entre le site Z et l'hémoglobine.

Le procédé de l'invention met avantageusement en oeuvre le composé de formule :

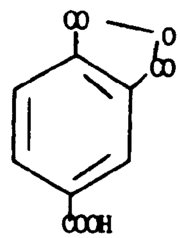

ou le composé de formule:

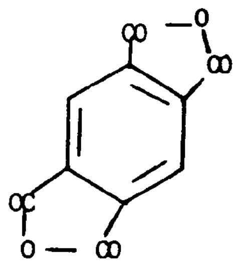

Lorsque le polymère est du dextrane, le milieu dans lequel est effectué la première étape, dont question ci-dessus, est de l'eau.

Dans le cas où le polymère est du polyéthylène glycol le milieu peut être de l'eau ou un milieu organique tel que le diméthylformamide.

Les liaisons covalentes entre le polymère et l'hémoglobine, sont établies entre les groupes $NH_2$ de l'hémoglobine et les groupes carboxyliques, aldéhyde ou OH, qui se trouvent sur les sites Z.

La liaison covalente entre le polymère et l'hémoglobine est une liaison amide, imine, amine ou carbamate.

Selon un mode de réalisation particulièrement avantageux de l'invention, les liaisons covalentes sont établies entre des groupes carboxyliques des sites Z du polymère et des groupes $NH_2$ de l'hémoglobine.

Selon un mode de réalisation avantageux, le procédé de l'invention concerne la préparation de conjugués d'hémoglobine à partir de dextrane aminé de masse moléculaire d'environ 40 000, et de $2.10^{-4}$ moles d'acide benzenehexacarboxylique par g de dextrane,

– les liaisons ioniques étant établies par l'intermédiaire des groupes carboxylates du benzenepentacarboxylate fixé sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

Selon un mode de réalisation avantageux, le procédé de l'invention concerne la préparation de conjugués d'hémoglobine à partir de dextrane aminé, de masse moléculaire d'environ 10 000, et de $3.5x10^{-4}$ moles d'acide benzenehexacarboxylique par g de dextrane,

– les liaisons ioniques étant établies par l'intermédiaire des groupes carboxylates du benzenepentacarboxylate fixés sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

Selon un mode de réalisation avantageux, le procédé de l'invention concerne la préparation de conjugués d'hémoglobine à partir de dextrane aminé, de masse moléculaire d'environ 10 000, et de $3,2x10^{-4}$ moles de benzenetetracarboxylique par g de dextrane,

– les liaisons ioniques étant établies par l'intermédiaire des groupes carboxylates du benzenetricarboxylate fixé sur le polymère, non engagés dans la liaison entre le polymère et l'acide benzenetetracarboxylique,

– et les liaisons covalentes étant établies entre les autres groupes carboxyliques de l'acide benzenetricarboxylique - à la fois non engagés dans la liaison entre l'acide benzenetetracarboxylique et le polymère et non engagés dans les liaisons ioniques ci-dessus définies - et des groupes $NH_2$ de l'hémoglobine.

Selon un mode de réalisation avantageux, le procédé de l'invention concerne la préparation de conjugués

d'hémoglobine à partir de dextrane aminé, de masse moléculaire d'environ 10 000, et de $4.10^{-4}$ moles de butane tetracarboxylique par g de polymère,

– les liaisons ioniques étant établies par l'intermédiaire des groupes carboxylates du butanetricarboxylate fixé sur le polymère, non engagés dans la liaison entre le polymère et l'acide butanetetracarboxylique

– et les liaisons covalentes étant établies entre les autres groupes carboxyliques de l'acide butanetricarboxylique - à la fois non engagés dans la liaison entre l'acide butanetetracarboxylique et le polymère et non engagés dans les liaisons ioniques ci-dessus définies - et des groupes $NH_2$ de l'hémoglobine.

Selon un mode de réalisation avantageux, le procédé de l'invention concerne la préparation de conjugués d'hémoglobine à partir de monométhoxypolyoyéthylène mono aminé, de masse moléculaire d'environ 5 000, et de $1.5x10^{-4}$ moles de benzenehexacarboxylique par g de polymère,

– les liaisons ioniques étant établies par l'intermédiaire des groupes carboxylates du benzenepentacarboxylate fixé sur le polymère, non engagés dans le liaison entre le polymère et l'acide benzenehexacarboxylique

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

Selon un mode de réalisation avantageux, le procédé de l'invention concerne la préparation de conjugués d'hémoglobine à partir de dextrane, de masse moléculaire d'environ 10 000, et de $6.3x10^{-4}$ moles de benzène-1,2,4-tricarboxylique par g de polymère,

– les liaisons ioniques étant établies par l'intermédiaire du groupe carboxylate du benzenedicarboxylate fixé sur le polymère, non engagé dans le liaison entre le polymère et l'acide benzene-1,2,4-tricarboxylique

– et les liaisons covalentes étant établies entre l'autre groupe carboxylique de l'acide benzènedicarboxylique - à la fois non engagé dans la liaison entre l'acide benzène-1,2,4-tricarboxylique et le polymère et non engagé dans les liaisons ioniques ci-dessus définies - et des groupes $NH_2$ de l'hémoglobine.

Selon un mode de réalisation avantageux, le procédé de l'invention concerne la préparation de conjugués d'hémoglobine à partir de dextrane, de masse moléculaire d'environ 10 000, et de $1.15x10^{-3}$ moles de benzène-1,2,4,5-tétracarboxylique par g de polymère,

– les liaisons ioniques étant établies par l'intermédiaire des groupes carboxylates du benzenetricarboxylate fixé sur le polymère, non engagés dans le liaison entre le polymère et l'acide benzene-1,2,4,5-tétracarboxylique

– et les liaisons covalentes étant établies entre les autres groupes carboxyliques de l'acide benzènetricarboxylique - à la fois non engagés dans la liaison entre l'acide benzène-1,2,4,5-tétracarboxylique et le polymère et non engagés dans les liaisons ioniques ci-dessus définies - et des groupes $NH_2$ de l'hémoglobine.

Les conjugués macromoléculaires obtenus par le procédé de l'invention présentent l'avantage d'être synthétisés facilement, sans formation ou presque de methémoglobine,(inférieure à 5 %) et de n'impliquer qu'un nombre limité de réactions sur l'hémoglobine, ce qui évite sa dénaturation.

Par ailleurs, les conjugués macromoléculaires obtenus par le procédé de l'invention ne renferment pas d'effecteurs de petite masse moléculaire, non liés, qui entraînent dans les expériences in vitro une augmentation de la $P_{50}$, mais qui peuvent être facilement éliminés par diffusion extrarénale ou extravasculaire au cours des essais in vivo, et dont l'effet s'annule rapidement.

Les conjugués macromoléculaires obtenus par le procédé de l'invention présentent l'avantage de n'avoir pour l'oxygène qu'une affinité modérée, et d'avoir un volume hydrodynamique élevé, ce qui pendant les opérations transfusionnelles augmente la persistance intravasculaire de l'hémoglobine par suppression de l'hémoglobinurie.

Les conjugués d'hémoglobine liés irréversiblement à des polymères polyanioniques préparés selon l'invention peuvent, lorsqu'ils sont dissous dans des solutions aqueuses de composition convenable, jouer le rôle de substituts du sang, notamment dans des interventions nécessitant des transfusions ou la perfusion d'organe.

L'invention vise par conséquent, également les solutions aqueuses renfermant les conjugués obtenus selon les procédés décrits ci-dessus et notamment les solutions rendues isotoniques du sang, par dialyse prolongée contre une solution de Tyrode ( de composition NaCl 8 g/l ; KCl 0,2 g/l ; $CaCl_2$ 0,2 g/l ; $MgCl_2$ 0,1g/l; $NaH_2PO_4$ 0,05 g/l ; $NaHCO_3$ 1 g/l ; D.glucose 1 g/l) par exemple et concentration par ultrafiltration jusqu'à obtenir pour l'hémoglobine, une concentration de 7 %.

Des préparations de conjugués macromoléculaires d'hémoglobine obtenus par le procédé selon l'invention, ont été examinées en tant que transporteurs d'oxygène potentiels. On a pu montrer qu'elles sont effectivement capables de fixer l'oxygène réversiblement et en particulier de le restituer plus facilement que l'hémoglobine libre ainsi que l'illustrent les courbes d'affinité pour l'oxygène des produits décrits dans les exemples 1, 3 et 5 ci-après et représentés sur la figure 5 qui sera commentée ci-dessous. Il apparaît en effet, que

ces préparations sont caractérisées par des pressions de demi-saturation ($P_{50}$) qui peuvent être très élevées (de 900 à 5 000 Pa) alors que dans les mêmes conditions (NaCl 0.05 M, pH 7, 25°C) celle de l'hémoglobine native est égale à 480 Pa.

Ainsi ces composés peuvent être utilisés pour fournir à des tissus ischémiés des quantités d'oxygène importantes. Ils peuvent également être utilisés en transfusion et être administrés à des patients, sous forme d'une solution aqueuse rendue isotonique du sang, en présence ou non d'excipients. Les composés peuvent aussi être lyophilisés en présence ou non d'un cryoprotecteur ou atomisés, et être redissous dans l'eau avant utilisation.

Les conjugués macromoléculaires obtenus par le procédé de l'invention ont été testés sur des souris relativement à la toxicité aiguë comme indiqué ci-après.

Les différents polymères polyanioniques décrits dans les exemples ont été injectés à des souris de souche SWISS dans les conditions suivantes : les polymères sont dissous dans de l'eau distillée à une concentration comprise entre 2,5 g et 5 g/l et le pH est ajusté à 7,4.

0,5 ml de chaque solution sont alors injectés par voie intrapéritonéale à 5 souris, dont le comportement est ensuite observé pendant une période de 7 jours. Ces essais n'ont permis de déceler aucun symptôme de toxicité aiguë.

L'invention a également pour objet de nouveaux conjugués macromoléculaires d'hémoglobine non biodégradables ou peu biodégradables, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce qu'il est constitué :

– d'une part par de l'hémoglobine, laquelle peut passer de façon réversible de la forme déoxygénée à la forme oxygénée,

– d'autre part par un polymère P hydrosoluble, non toxique, de préférence non antigénique, hémocompatible, de masse moléculaire d'environ 1 000 à environ 500 000, de préférence d'environ 1 000 à environ 100 000, comportant un ou plusieurs groupes polaires, de préférence des groupes hydroxyles, carboxyles ou amines,

– lequel polymère comporte des sites Z contenant d'une part au moins une charge négative portée par au moins un groupe choisi parmi les groupes suivants : sulfate, phosphate, carboxylate et destinée à créer une liaison ionique avec le polymère, et contenant d'autre part au moins un groupe carboxylique, aldéhyde ou OH, destiné à créer une liaison covalente avec le polymère,

– le polymère P étant relié à l'hémoglobine

. d'une part par l'intermédiaire d'au moins une liaison ionique établie entre l'une au moins charges négatives des sites Z portés par le polymère P et l'hémoglobine, et

. d'autre part par l'intermédiaire d'au moins une liaison covalente établie, entre l'un au moins des groupes carboxyliques, aldéhyde ou OH du susdit site Z porté par le polymère P et l'hémoglobine,

– le nombre de liaisons covalentes entre le polymère et l'hémoglobine étant tel que le conjugué macromoléculaire a une masse moléculaire moyenne d'environ 70 000 à environ 1 000 000, de préférence d'environ 70 000 à environ 500 000, la relation entre le nombre et la nature des charges négatives, le nombre de sites et le nombre de monomères étant la suivante :

– lorsqu'il s'agit de site Z contenant un groupe anionique unique constitué d'un sulfate ou d'un phosphate, il y a au moins un tel site Z par chaîne de polymère et au plus un tel site Z tous les onze monomères,

– lorsqu'il s'agit de site Z contenant un groupe anionique unique constitué d'un carboxylate, non engagé dans la liaison entre le site et le polymère et non engagé dans la liaison covalente entre le polymère et l'hémoglobine, il y au moins un tel site Z par chaîne de polymère,

– lorsqu'il s'agit de site Z contenant deux charges anioniques provenant de deux carboxylates, il y a au moins un tel site Z par chaîne de polymère et au plus un tel site Z tous les six monomères.

Cette classe de conjugués macromoléculaires nouveaux de l'invention se caractérise notamment :

– par le fait que l'une au moins des liaisons ioniques et l'une au moins des liaisons covalentes sont établies à partir de groupes respectifs appropriés situés sur le même site Z

– et par le fait qu'il y a au moins un seul site Z sus-défini par chaîne de polymère ; en d'autres termes, qu'un seul site Z sus-défini par chaîne suffit.

On a constaté que cette classe de nouveaux conjugués macromoléculaires présente des propriétés oxyphoriques intéressantes, alors que les conjugués macromoléculaires d'hémoglobine dans lesquels les conditions entre la nature, le nombre de charges anioniques sur un site Z, et le nombre de sites Z par chaîne de polymère sont les mêmes mais différent des conjugués définis ci-dessus par le fait que la liaison covalente est établie entre un groupe carboxylique, aldéhyde ou OH qui n'est pas situé sur le site Z (qui comporte le groupe anionique), présentent une activité oxyphorique peu ou pas intéressante.

Selon un mode de réalisation avantageux de l'invention, tous les sites Z qui interviennent dans les liaisons

covalentes entre le polymère et l'hémoglobine sont les mêmes que les sites Z qui interviennent dans les liaisons ioniques entre le polymère et l'hémoglobine.

Une classe avantageuse de conjugués macromoléculaires d'hémoglobine selon l'invention est constituée par ceux dans lesquels les liaisons covalentes entre le polymère P et l'hémoglobine sont établies entre au moins un groupe carboxylique ou aldéhyde ou OH porté par les sites Z et au moins une amine de l'hémoglobine située dans le site allostérique de l'hémoglobine, notamment l'amine de l'une au moins des deux valines β-terminales de l'hémoglobine.

Une autre classe avantageuse de conjugués macromoléculaires d'hémoglobine selon l'invention est constituée par ceux dans lesquels les ponts salins entre les groupes $NH_3^+$ et les groupes $COO^-$ internes de l'hémoglobine sont intacts lorsque l'hémoglobine est sous forme déoxygénée.

Une autre classe avantageuse de conjugués macromoléculaires selon l'invention est constituée par ceux dans lesquels le polymère P est choisi parmi les polysaccharides, notamment les hydroxyalkylamidons dont la radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkylène glycols dans lesquels le groupe alkylène comporte de 2 à 5 atomes de carbone, notamment le polyéthylène glycol et le polypropylène glycol.

Une autre classe avantageuse de conjugués macromoléculaires selon l'invention est constituée par ceux dans lesquels le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

Une autre classe avantageuse de conjugués macromoléculaires selon l'invention est constituée par ceux dans lesquels le site Z est relié au polymère par l'intermédiaire d'une fonction ester, éther, amide ou amine.

Une autre classe avantageuse de conjugués macromoléculaires selon l'invention est constituée par ceux dans lesquels le site Z comporte des groupes $OSO_3H$, $OPO_3H_2$, $O-CH(COOH)_2$, $-O-CH(COOH)-CH_2-COOH$,

$$O-CH_2-\left[(\underset{COOH}{\underset{|}{CH}}-CH_2)\right]_n-CH_2-COOH,$$

n variant de 1 à environ 4, ou provient du pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3 diphosphoglycérate.

Une autre classe avantageuse de composés selon l'invention est constituée par ceux dans lesquels aucune liaison ionique et aucune liaison covalente n'est susceptible de s'établir ailleurs qu'entre les groupes respectifs appropriés des sites Z du polymère et l'hémoglobine.

Une autre classe avantageuse de conjugués macromoléculaires selon l'invention est constituée par ceux dans lesquels les liaisons covalentes entre le polymère et l'hémoglobine sont établies à partir des groupes carboxyliques provenant des sites Z fixés sur le polymère et des groupes $NH_2$ de l'hémoglobine.

Une autre classe avantageuse de conjugués macromoléculaires selon l'invention est constituée par ceux dans lesquels les liaisons ioniques entre le polymère P et l'hémoglobine sont établies entre les groupes carboxylates des sites Z et l'hémoglobine.

Tout ce qui a été dit à propos des conjugués macromoléculaires obtenus à partir du nouveau procédé décrit ci-dessus, s'applique notamment aux conjugués macromoléculaires de l'invention.

Les nouveaux conjugués macromoléculaires selon l'invention peuvent être obtenus soit à partir d'un procédé mettant en oeuvre l'oxyhémoglobine, soit à partir d'un procédé mettant en oeuvre la déoxyhémoglobine.

Les conjugués d'hémoglobine selon l'invention peuvent être obtenus de la façon suivante :

– on fixe, dans une première étape, des sites Z comportant les groupes ioniques destinés à créer une liaison ionique avec l'hémoglobine sur un polymère P, dans le rapport suivant :

– lorsqu il s'agit de site Z contenant un groupe anionique unique constitué d'un sulfate ou d'un phosphate, il y a au moins un tel site Z par chaîne de polymère et au plus un tel site Z tous les onze monomères,

– lorsqu'il s'agit de site Z contenant un groupe anionique unique constitué d'un carboxylate, non engagé dans la liaison entre le site et le polymère et non engagé dans la liaison covalente entre le polymère et l'hémoglobine, il y au moins un tel site Z par chaîne de polymère,

– lorsqu'il s'agit de site Z contenant deux charges anioniques provenant de deux carboxylates non engagés dans la liaison entre le site et le polymère et non engagé dans la liaison covalente entre le polymère et

l'hémoglobine, il y a au moins un tel site Z par chaîne de polymère et au plus un tel site Z tous les six monomères,
le polymère P étant hydrosoluble, non toxique, de préférence non antigénique, hémocompatible, de masse moléculaire d'environ 1 000 à environ 500 000, de préférence d'environ 1 000 à environ 100 000, comportant des groupes polaires, de préférence des groupes hydroxyles, carboxyliques ou amines, et les sites Z contenant d'une part au moins une charge négative portée par des groupes, sulfates et/ou phosphates et/ou carboxylates, et contenant d'autre part au moins un groupe carboxylique, aldéhyde ou OH,

. soit en utilisant un composé Z-Y dans lequel Y est une fonction active ou activable, telle que aldéhyde, carboxylique, amine, hydroxyle ou halogène, ou bien

. soit en effectuant un greffage radicalaire des sites Z sur le polymère P ;

– puis dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris de 5 à 9,

pour former d'une part au moins une liaison ionique entre l'un au moins des sites Z portés par le polymère et l'hémoglobine et d'autre part au moins une liaison covalente entre le même susdit site Z portés par le polymère et l'hémoglobine,

– lorsque la réaction ci-dessus indiquée conduit éventuellement à des fonctions imines, celles-ci peuvent être stabilisées en fonctions amines, par exemple par réduction à l'aide de $NaBH_4$, $NaCNBH_3$, le diméthylaminoborane ou HCOOH.

Les conjugués selon l'invention peuvent également être obtenus selon le procédé décrit dans la demande France n° 2 600 894.

Les conjugués d'hémoglobine liés irréversiblement à des polymères polyanioniques préparés selon l'invention peuvent, lorsqu'ils sont dissous dans des solutions aqueuses de composition convenable, jouer le rôle de substituts du sang, notamment dans des interventions nécessitant des transfusions ou la perfusion d'organe.

L'invention vise par conséquent, également les solutions aqueuses renfermant les conjugués décrits ci-dessus et notamment les solutions rendues isotoniques du sang, par dialyse prolongée contre une solution de Tyrode ( de composition NaCl 8 g/l ; KCl 0,2 g/l ; $CaCl_2$ 0,2 g/l ; $MgCl_2$ 0,1 g/l ; $NaH_2PO_4$ 0,05 g/l ; $NaHCO_3$ 1 g/l ; D.glucose 1 g/l) par exemple et concentration par ultrafiltration jusqu à obtenir pour l'hémoglobine, une concentration de 7 %.

Des préparations de conjugués macromoléculaires d'hémoglobine obtenus selon l'invention, ont été examinées en tant que transporteurs d'oxygène potentiels. On a pu montrer qu'elles sont effectivement capables de fixer l'oxygène réversiblement et en particulier de le restituer plus facilement que l'hémoglobine libre. Il apparaît en effet, que ces préparations sont caractérisées par des pressions de demi-saturation ($P_{50}$) qui peuvent être très élevées (de 900 à 5 000 Pa) alors que dans les mêmes conditions (NaCl 0.05 M, pH 7, 25°C) celle de l'hémoglobine native est égale à 430 Pa.

Ainsi ces conjugués peuvent être utilisés pour fournir à des tissus ischémiés des quantités d'oxygène importantes. Ils peuvent également être utilisés en transfusion et être administrés à des patients, sous forme d'une solution aqueuse rendue isotonique du sang, en présence ou non d'excipients. Les composés peuvent aussi être lyophilisés en présence ou non d'un cryoprotecteur ou atomisés, et être redissous dans l'eau avant utilisation.

Les conjugués macromoléculaires selon l'invention ont été testés relativement à la toxicité aiguë.

L'invention sera mieux comprise à l'aide des exemples qui suivent ci-après donnés à titre non limitatif.

EXEMPLE 1 : Synthèse d'un conjugué covalent d'hémoglobine et de dextrane-benzènepentacarboxylate (M.M du dextrane 40000). Couplage avec l'oxyhémoglobine.

Du dextrane aminé contenant $5.10^{-4}$ moles de $NH_2$ par g de produit sec (soit 8 moles de $NH_2$ pour 100 moles de glucopyranose) est préparé selon P. HUBERT et al, Proc. Natl. Acad. Sci. USA 1978, 75, 3143, par action de l'ammoniaque sur du dextrane activé à l'épichlorhydrine.

1 g de ce dextrane aminé est dissous dans 20 ml d'eau et le pH est ajusté à 6,5 avec HCl 0.1N. On ajoute ensuite 1,7 g d'acide benzène hexacarboxylique, puis 1 g de chlorhydrate de N'-éthyl-N-(3-diméthylaminopropyl) carbodiimide (EDCI). Le pH est ramené à 6,5 et la réaction est poursuivie pendant 3 jours à 20° C. Après dialyse contre une solution d'acétate de sodium 0,5 M, le mélange est traité à l'anhydride acétique pour bloquer les fonctions amines non substituées du dextrane. La solution est alors épurée des contaminants de petite masse moléculaire par dessalage sur une colonne d'Ultrogel AcA 202 (IBF-France) avec un tampon phosphate 0,2 M, pH 7,2.

Après une dialyse prolongée contre de l'eau, la solution contenant le polymère polyanionique est lyophilisée. Le composé est conservé au froid sous vide.

Le polycarboxylate de dextrane obtenu contient $2.10^{-4}$ moles de benzene-pentacarboxylate (B.P.C.) par g de polymère.

5 g du polycarboxylate de dextrane sont dissous dans 250 ml de NaCl 0,05 M. On ajuste le pH à 7 avec de la soude 0.1M et on ajoute 150 ml d'une solution d'hémoglobine à 10 %. On ajoute ensuite 600 mg de chlorhydrate de N′-éthyl-N-(3-diméthyl-aminopropyl) carbodiimide (EDCI) et la réaction est poursuivie à 20°C pendant 2 heures. On vérifie qu'il n'y a plus d'hémoglobine libre sur le chromatogramme obtenu sur une colonne d'Ultrogel AcA 34 (IBF France). La $P_{50}$ du conjugué est de 2660 Pa (25°C, Tris 0,05 M, pH = 7,2 ; Hb libre dans les mêmes conditions : $P_{50}$ = 430 Pa).

EXEMPLE 2 : Synthèse d'un conjugué covalent d'hémoglobine et de dextrane-benzènepentacarboxylate (M.M du dextrane 10000). Couplage avec l'oxyhémoglobine.

Le polymère est préparé à partir d'un dextrane aminé contenant $4.10^{-4}$ moles de $NH_2$ par g de produit sec, préparé de la même façon que celui de l'exemple 1.

1 g de ce dextrane aminé est dissous dans 20 ml d'eau et le pH est ajusté à 7,5 avec NaOH 0,1M.

On ajoute ensuite 0,7 g d'acide benzène hexacarboxylique (B.H.C.) puis 1 g de chlorhydrate de N′-ethyl-N-(3-diméthyl-aminopropyl)-carbodiimide (EDCI). La réaction est poursuivie pendant 1 jour à 20°C. Après dialyse contre une solution d'acétate de sodium 0.5 M le mélange est traité à l'anhydride acétique pour bloquer les fonctions amines non substituées du dextrane. La solution est alors épurée des contaminants de petite masse moléculaire par dessalage sur une colonne d'Ultrogel AcA 202 (IBF France) avec un tampon phosphate 0,2 M, pH = 7,2. On vérifie par chromatographie liquide haute performance à l'aide d'une colonne TSK G 3000 SW (LKB France) que le polycarboxylate de dextrane est totalement épuré du benzène hexacarboxylate en excès.

Après une dialyse prolongée contre de l'eau la solution contenant le polymère polyanionique est lyophilisée. Le composé est conservé au froid sous vide. Le polycarboxylate de dextrane obtenu contient 3,5 $10^{-4}$ moles de B.P.C. par g de polymère.

2,5 g de polycarboxylate de dextrane sont dissous dans 150 ml de NaCl aqueux 0,05 M. On ajoute 100 ml d'une solution d'hémoglobine à 10 %. On ajuste le pH à 7,5 avec de la soude 0,1 M et on complète à 300 ml avec NaCl aqueux 0,05 M.

On ajoute ensuite 350 mg d'EDCI et la réaction est poursuivie à 20°C pendant 2 heures. On vérifie qu'il n'y a plus d'hémoglobine libre sur le chromatogramme obtenu sur une colonne TSK G 3000 SW (LKB France).

La Figure 2 représente la densité optique en fonction du volume d'élution et permet de constater qu'il n'y a plus d'hémoglobine libre. Sur cette figure, Vo correspond au volume exclus de la colonne et la flèche sous Hb correspond au volume d'élution de l'hémoglobine libre, c'est-à-dire non couplée au polymère.

La $P_{50}$ du conjugué est de 2720 Pa (25°C, Tris 0,05M, pH = 7,2 ; Hb libre dans les mêmes conditions : $P_{50}$ = 430 Pa).

EXEMPLE 3 : Synthèse d'un conjugué covalent d'hémoglobine et de dextrane-benzènetricarboxylate (M.M du dextrane 10 000). Couplage à l'oxyhémoglobine.

Le polymère est préparé à partir d'un dextrane aminé contenant $4.10^{-4}$ moles de $NH_2$ par g de polymère sec.

1 g de ce polymère aminé est dissous dans 20 ml d'eau et le pH est ajusté à 7,5 avec NaOH 0,1M. On ajoute ensuite 0,5 g d'acide benzène-1,2,4,5-tétracarboxylique (B.T.C) puis 0,6 g d'EDCI. La réaction est poursuivie pendant 1 jour à 20°C. La solution est alors traitée comme dans l'exemple 2.

Le polycarboxylate de dextrane obtenu contient 3,2 $10^{-4}$ moles de benzenetricarboxylate par g de polymère.

0,5 g de ce polycarboxylate de dextrane sont dissous dans 25 ml de NaCl aqueux 0,05 M. On ajoute 10 ml d'une solution d'hémoglobine à 10 %. On ajuste le pH à 7,5 avec de la soude 0,1M et on complète à 40 ml avec NaCl aqueux 0,05 M.

On ajoute ensuite 50 mg d'EDCI et la réaction est poursuivie à 20°C pendant 2 heures. On vérifie qu'il n'y a plus d'hémoglobine libre sur le chromatogramme obtenu sur une colonne TSK G 3000 SW (LKB France). La Figure 3 représente la densité optique en fonction du volume d'élution et permet de constater qu il n'y a plus d'hémoglobine libre. Sur cette figure, la flèche sous Vo correspond au volume exclus de la colonne et la flèche sous Hb correspond au volume d'élution de l'hémoglobine libre, c'est-à-dire non couplée.

La $P_{50}$ du conjugué est de 710 Pa (25°C, Tris 0,05 M, pH = 7,2 ; Hb libre dans les mêmes conditions, $P_{50}$

= 430 Pa).

EXEMPLE 4 : Synthèse d'un conjugué covalent d'hémoglobine est dextrane-butanetricarboxylate (M.M du dextrane 10 000). Couplage à l'oxyhémoglobine.

Le polymère est préparé de la même façon que celui de l'exemple 3 en utilisant l'acide n-butane 1,2,3,4-tétracarboxylique (Bu.T.C.). Le polycarboxylate de dextrane obtenu contient $4.10^{-4}$ moles de butanetricarboxylate par g de polymère.

0,6 g de ce polycarboxylate de dextrane sont dissous dans 25 ml de NaCl aqueux 0,05 M. On ajoute 10 ml d'une solution d'hémoglobine à 10 %. On ajuste le pH à 7,5 avec de la soude 0,1 M et on complète à 40 ml avec NaCl aqueux 0,05 M.

On ajoute ensuite 70 mg d'EDCI et la réaction est poursuivie à 20°C pendant 2 heures. On vérifie qu'il n'y a plus d'hémoglobine libre sur le chromatogramme obtenu sur une colonne TSK G 3000 SW (LKB France). La $P_{50}$ du conjugué obtenu est 540 Pa. (25°C, Tris 0,05 M, pH = 7,2 ; hémoglobine libre dans les mêmes conditions, $P_{50}$ = 430 Pa).

EXEMPLE 5 : Synthèse d'un conjugué covalent d'hémoglobine et de monométhoxypolyoxyéthylène-benzène pentacarboxylate (M.M du polyoxyéthylène : 5000. Couplage avec l'oxyhémoglobine.

Du monométhoxypolyoxyéthylène aminé, ($MPOE-NH_2$, 2 $10^{-4}$ moles de $NH_2$ par g de produit sec) est préparé selon M. LEONARD et al, Tetrahedron 1984, 40, 1581, par action du bromure de thionyle sur du MPOE, suivie d'une substitution par l'ammoniac.

2 g de ce $MPOE-NH_2$ sont dissous dans 50 ml d'eau et le pH est ajusté à 8. On ajoute ensuite 1.4 g d'acide benzène hexacarboxylique (B.H.C), puis 0,8 g de chlorhydrate de N'-éthyl-N (3-diméthyl-aminopropyl) carbodiimide (EDCI). On laisse la réaction se poursuivre une nuit à température ambiante. Le mélange est ensuite acidifié à pH 1 par HCl aqueux 1M ; le polymère est extrait au chlorure de méthylène, puis précipité par l'éther anhydre.

Il est ensuite purifié par chromatographie sur colonne de Dowex, et ensuite sur colonne d'Ultrogel d'AcA 202 (IBF, France). L'éluat est dialysé contre de la soude aqueuse (pH 9), puis le polymère est lyophilisé.

0,2 g de ce polymère contenant environ 1,5 $10^{-4}$ moles de benzène-pentacarboxylate (B.P.C) par g de polymère sont dissous dans 10 ml d'eau. On ajuste le pH à 7 et on ajoute 10 ml d'une solution d'hémoglobine à 10 %, puis 10 mg d'EDCI. Après une heure de réaction, le mélange est chromatographié sur colonne d'Ultrogel AcA 54 (IBF, France) afin de purifier le conjugué polymère d'hémoglobine. La figure 4 correspond à la variation de la densité optique en fonction du volume d'élution et permet de constater qu'il n'y a plus d'hémoglobine libre. Vo correspond au volume exclus de la colonne ; la flèche sous Hb correspond au volume d'élution de l'hémoglobine libre, c'est-à-dire non couplée, et celle sous MPOE-BPC correspond au volume d'élution du polymère non lié à l'hémoglobine.La $P_{50}$ de ce conjugué est de 2400 Pa (25°C, Tris 0.05 M, pH 7,2 ; Hb libre dans les mêmes conditions : $P_{50}$ = 430 Pa).

EXEMPLE 6

Synthèse d'un conjugué covalent d'hémoglobine et de dextrane-benzènedicarboxylate (MM du dextrane 10 000 : liaison ester entre le dextrane et le site polycarboxylate). Couplage à l'oxyhémoglobine.

Le dextrane-benzènedicarboxylate est préparé en faisant réagir 7.5g de benzène 1,2,4 tricarboxylique anhydride avec 32 g de dextrane en milieu aqueux à pH 9 pendant 15 h; La solution est ensuite débarrassée de l'acide benzène 1,2,4 tricarboxylique résiduel par dessalage sur une colonne d'Ultrogel AcA 202 (IBF-France) avec un tampon phosphate 0,2M, pH 7.2.

Après une dialyse prolongée contre de l'eau, la solution contenant le polymère est lyophilisée. le polycarbolate de dextrane contient 6,3 $10^{-4}$ moles de benzènedicarboxylate (B.D.C) par g de polymère.

Ceci correspond à environ 1 site Z comportant deux groupes anioniques constitués de deux groupes carboxylates tous les 10 monomères.

11 g de polycarboxylate de dextrane sont dissous dans 250 ml d'eau. On ajuste le pH à 6.5 et on ajoute 150 ml d'une solution d'hémoglobine à 10 %. On ajoute ensuite 160 mg de chorhydrate de N'-éthyl-N (3 diméthylaminopropyl) carbodiimide (EDCI) et la réaction est poursuivie à 20°C pendant 2 heures.

Le conjugué d'hémoglobine obtenu est un conjugué nouveau.

Le mélange est chromatographié sur Ultrogel AcA 54 (IBF France) pour éliminer l'hémoglobine libre et les coontaminants. La $P_{50}$ du conjugué purifié est de 870 Pa (25°C, Tris 0.05M, pH 7.2; Hb libre dans les mêmes conditions: $P_{50}$ = 430 Pa).

EXEMPLE 7:

Synthèse d'un conjugué covalent d'hémoglobine et de dextrane-benzènetricarboxylate (MM du dextrane 10 000 - liaison ester entre le dextrane et le site polycarboxylate). Couplage à l'oxyhémoglobine.

Le dextrane-benzènetricarboxylate est préparé en faisant réagir 26 g de benzène 1,2,4,5 tétracarboxylique dianhydride avec 20 g de dextrane en milieu aqueux à pH 9 pendant 15 h; La solution est ensuite débarrassée de l'acide benzène 1,2,4,5 tetracarboxylique résiduel par dessalage sur une colonne d'Ultrogel AcA 202 (IBF-France) avec un tampon phosphate 0,2M, pH 7.2.

Après une dialyse prolongée contre de l'eau, la solution contenant le polymère est lyophilisée. Le polycarboxylate de dextrane conient $1,15.10^{-3}$ moles de benzènetricarboxylate (B.T.C.) par g de polymère.

5 g de polycarboxylate de dextrane sont dissous dans 250 ml d'eau. On ajuste le pH à 7.0 et on ajoute 150 ml d'une solution d'hémoglobine à 10%. On ajoute ensuite 120 mg de chlorhydrate de N'-éthyl-N(3 diméthylaminopropyl)-carbodiimide (EDCI) et la réaction est poursuivie à 20°C pendant 2 heures.

Le mélange est chromatographié sur Ultrogel AcA 54 (IBF France) pour éliminer l'hémoglobine libre et les contaminants. La $P_{50}$ du conjugué purifié est de 2000 Pa (25°C, Tris 0,05M, pH 7.2; Hb libre dans les mêmes conditions: $P_{50}$ = 430 Pa).

On peut également préparer le conjugué défini ci-dessus à l'aide de dextrane et d'acide benzène 1,2,4,5 tétracarboxylique, en présence de carbodiimide soluble dans l'eau tel que le chlorhydrate de N'-éthyl-N(3 diméthylaminopropyl) carbodiimide (EDCI).

EXEMPLE 8:

On prépare, selon le protocole décrit dans l'exemple 5, les conjugués :

. d'hémoglobine et de polyéthylèneglycol-benzènepentacarboxylique
. d'hémoglobine et de polyéthylèneglycol benzenetétracarboxylique
. d'hémoglobine et de polyéthylène-glycol-benzènetricarboxylique
. d'hémoglobine et de polyéthylèneglycol-benzène-dicarboxylique.

EXEMPLE 9:

On prépare, selon le protocole décrit dans les exemples 6 et 7, le conjugué d'hémoglobine et de polyéthylèneglycol-benzènetricarboxylique.

Pour préparer le polyéthylèneglycol-benzènetricarboxylique, on fait réagir du benzene-tétracarboxylique dianhydride avec du polyéthylèneglycol en milieu organique, tel que diméthylformamide. La solution est ensuite débarrassée de l'acide benzène tetracarboxylique résiduel par exemple par dessalage sur une colonne d'Ultrogel AcA 202 (IBF-France) avec un tampon phosphate 0,2M,pH 7.2.

Après une dialyse prolongée contre de l'eau, la solution contenant le polymère est lyophilisée.

Le polycarboxylate de polyéthylèneglycol est dissous dans de l'eau. On ajuste le pH à 7.0 et on ajoute une solution d'hémoglobine, par exemple à 10%. On ajoute ensuite du chlorhydrate de N'-éthyl-N(3 diméthylaminopropyl)-carbodiimide (EDCI) et la réaction est poursuivie à 20°C pendant 2 heures.

Le mélange est chromatographié sur Ultrogel AcA 54 (IBF France) pour éliminer l'hémoglobine libre et les contaminants.

On prépare également selon le protole indiqué précedemment, le conjugué d'hémoglobine et de polyéthylèneglycol-benzène-dicarboxylique (à l'aide de polyéthylèneglycol et de benzène 1,2,4 tricarboxylique anhydride).

On peut également préparer des polyéthylèneglycols - benzènepolycarboxylates en faisant réagir les acides benzènepolycarboxyliques sur les fonctions OH du polymère en présence de dicyclohexylcarbodiimide/diméthylaminopyridine dans le diméthylformamide.

EXEMPLE COMPARATIF 1 :

Cet exemple a pour but de comparer la $P_{50}$ obtenue en mettant en oeuvre le procédé de l'invention pour préparer des conjugués d'hémoglobine dans lesquels la liaison covalente est établie entre un groupe aldéhyde non porté par le site Z sur lequel se trouve le groupe anionique phosphate.

On prépare du phosphate de dextrane à l'aide de dextrane de départ de masse moléculaire d'environ 10 000 que l'on transforme en phosphate disodique contenant 8% de phosphore, conformément au protocole décrit dans l'exemple 1 de la demande France n° 2 600 894.

On active le phosphate de dextrane par le périodate de sodium de façon à obtenir 13 aldéhydes pour 100

unités glucosidiques.

Ce dextrane phosphate aldéhydique est mis à réagir avec l'hémoglobine dans la proportion 1.5 en masse (masse de dextrane / masse d'hémoglobine), à pH 8; Orla réaction est poursuivie à 4°C pendant 24 h. On ajoute alors une solution de $NaBH_4$ dans NaOH $10^{-3}N$. Le mélange est chromatographié pour contrôler qu'il n'y a plus d'hémoglobine libre (HPLC sur colonne TSK G 3000 SW Beckman).

Quand la réaction est faite sur la déoxyhémoglobine selon le procédé décrit dans la demande France n° 2 600 894, la $P_{50}$ du conjugué d'hémoglobine est de 3380 Pa (25°C, Tris 0,05 M, pH 7.2).

Dans les mêmes conditions, la $P_{50}$ de l'hémoglobine libre est de 450 Pa.

Quand la réaction est faite sur l'oxyhémoglobine, conformément au procédé de la présente invention, la $P_{50}$ du conjugué d'hémoglobine est de 425 Pa (25°C, Tris 0,05 M, pH 7.2).

Dans les mêmes conditions, la $P_{50}$ de l'hémoglobine libre $P_{50}$ est de 430 Pa.

Cet exemple comparatif montre que le procédé de préparation de conjugués d'hémoglobine à l'aide d'hémoglobine oxygénée n'est pas applicable lorsque la liaison covalente est établie entre un groupe aldéhyde (carboxylique ou OH) non fixé sur le site Z comportant les charges anioniques.

## Revendications

### Revendications pour les Etats contractants suivants : AT, BE, CH, DE, FR, GB, GR, IT, LI, LU, NL, SE

1. Procédé de préparation de conjugués macromoléculaire hydrosolubles d'hémoglobine, non biodégradables ou peu biodégradables, pendant le temps durant lequel le conjugué macromoléculaire doit assurer des fonctions oxyphoriques dans la plasma, présentant pour l'oxygène une affinité inférieure à celle de l'hémoglobine libre, caractérisé en ce que :

– on fixe, dans une première étape, des sites Z sur un polymère P, à raison d'au moins un site Z par chaîne de polymère, le polymère P étant hydrosoluble, non toxique, de préférence non antigénique, hémocompatible, de masse moléculaire d'environ 1 000 à environ 500 000, de préférence d'environ 1 000 à environ 100 000, comportant un ou plusieurs groupes polaires, de préférence des groupes hydroxyles, carboxyliques ou amines, et les sites Z contenant d'une part au moins une charge négative portée par des groupes, sulfates et/ou phosphates et/ou carboxylates et destinée à créer une liaison ionique avec l'hémoglobine, et contenant d'autre part au moins un groupe carboxylique, aldéhyde ou OH, destiné à créer une liaison covalente avec l'hémoglobine,

. soit en utilisant un composé Z-Y dans lequel Y est une fonction active ou activable à l'aide d'un agent d'activation, telle que aldéhyde, carboxylique, amine, hydroxyle ou halogène, ou bien

. soit en effectuant un greffage radicalaire des sites Z sur le polymère P;

– puis dans une seconde étape, on fait réagir le polymère P comportant le ou les sites Z avec de l'hémoglobine sous oxygénée dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris d'environ 5 à environ 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine,

– lorsque la réaction ci-dessus indiquée conduit éventuellement à des fonctions imines, celles-ci peuvent être stabilisées en fonctions amines, par exemple par réduction à l'aide de $NaBH_4$, $NaCNBH_3$, le diméthylaminoborane ou HCOOH.

2. Procédé de préparation de conjugués macromoléculaires selon la revendication 1, caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et l'hémoglobine) soit la suivante :

– lorsque chaque site Z contient un groupe anionique unique constitué d'un sulfate ou d'un phosphate, il y a au moins un tel site Z tous les dix monomères du polymère,

– lorsqu'il s'agit de site Z contenant au moins deux groupes anioniques, constitués de sulfates et/ou de phosphates, il y a au moins un tel site Z par chaîne de polymère,

– lorsque chaque site Z contient des charges anioniques provenant de carboxylates, il faut au moins deux groupes carboxylates sur un même site Z, et au moins un tel site Z tous les cinq monomères,

– lorsque l'un au moins des sites Z contient au moins trois charges négatives provenant de carboxylates,

il y a au moins un tel site par chaîne de polymère.

3. Procédé de préparation de conjugués macromoléculaires selon la revendication 1, caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et l'hémoglobine) soit la suivante :

– lorsqu'il s'agit de site Z contenant un groupe anionique unique constitué d'un sulfate ou d'un phosphate, il y a au moins un tel site Z par chaîne de polymère et au plus un site Z tous les onze monomères,

– lorsqu'il s'agit de site Z contenant un groupe anionique unique constitué d'un carboxylate, non engagé dans la liaison entre le site et le polymère et non engagé dans la liaison covalente entre le polymère et l'hémoglobine, il y au moins un tel site Z par chaîne de polymère,

– lorsqu'il s'agit de site Z contenant deux charges anioniques provenant de deux carboxylates non engagés dans la liaison entre le site et le polymère et non engagé dans la liaison covalente entre le polymère et l'hémoglobine, il y a au moins un site Z par chaîne de polymère et au plus un tel site Z tous les six monomères.

4. Procédé selon les revendications 1 à 3, caractérisé en ce que les liaisons covalentes entre le polymère P et l'hémoglobine sont établies entre au moins un groupe carboxylique, aldéhyde ou OH porté par les sites Z et au moins une amine de l'hémoglobine située dans le site allostérique de l'hémoglobine lorsque celle-ci est sous forme déoxygénée, notamment l'amine de l'une au moins des deux valines β-terminales de l'hémoglobine.

5. Procédé selon les revendications 1 à 4, caractérisé en ce que les ponts salins entre les groupes $NH_3^+$ et les groupes $COO^-$ internes de l'hémoglobine sont intacts lorsque l'hémoglobine est sous forme déoxygénée.

6. Procédé selon les revendications 1 à 5, caractérisé en ce que le polymère P est choisi parmi les polysaccharides, notamment les hydroxyalkylamidons dont la radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkylène glycols dans lesquels le groupe alkylène comporte de 2 à 5 atomes de carbone, notamment le polyéthylène glycol et le polypropylène glycol.

7. Procédé selon les revendications 1 à 6, caractérisé en ce que le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

8. Procédé selon les revendications 1 à 7, caractérisé en ce que le site Z est relié au polymère par l'intermédiaire d'une fonction ester, éther, amide ou amine.

9. Procédé selon les revendications 1 à 8, caractérisé en ce que le site Z comporte les groupes $OSO_3H$, $OPO_3H_2$, $O-CH(COOH)_2$, $-O-CH(COOH)-CH_2-COOH$,

$$O-CH_2-\left[(\underset{COOH}{\underset{|}{CH}}-CH_2)\right]_n-CH_2-COOH,$$

n variant de 1 à environ 4, ou provient du pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3-diphosphoglycérate.

10. Procédé selon les revendications 1 à 9, caractérisé en ce que les liaisons covalentes entre le polymère et l'hémoglobine sont établies à partir des groupes carboxyliquees provenant des sites Z fixés sur le polymère et des groupes $NH_2$ de l'hémoglobine.

11. Procédé selon les revendications 1 à 10, caractérisé en ce que les liaisons ioniques entre le polymère P et l'hémoglobine sont établies entre les groupes carboxylates des sites Z et l'hémoglobine.

12. Procédé selon la revendication 1, caractérisé en ce que le polymère est obtenu à partir de dextrane aminé de masse moléculaire d'environ 40 000, et de $2.10^{-4}$ moles d'acide benzenehexacarboxylique par g de dextrane,

– les liaisons ioniques étant établies par l'intermédiaire des groupes carboxylates du benzenepentacarboxylate fixé sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

13. Procédé selon la revendication 1, caractérisé en ce que le polymère est obtenu à partir du dextrane aminé, de masse moléculaire d'environ 10 000, et de $3.5x10^{-4}$ moles d'acide benzenehexacarboxylique par g de dextrane,

– les liaisons ioniques étant établies par l'intermédiaire des groupes carboxylates du benzenepentacarboxylate fixés sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

14. Procédé selon la revendication 1, caractérisé en ce que le polymère est obtenu à partir de dextrane aminé, de masse moléculaire d'environ 10 000, et de $3,2x10^{-4}$ moles de benzenetetracarboxylique par g de dextrane,

– les liaisons ioniques étant établies par l'intermédiaire des groupes carboxylates du benzenetricarboxylate fixé sur le polymère, non engagés dans la liaison entre le polymère et l'acide benzenetetracarboxylique,

– et les liaisons covalentes étant établies entre les autres groupes carboxyliques de l'acide benzenetricarboxylique - à la fois non engagés dans la liaison entre l'acide benzenetetracarboxylique et le polymère et non engagés dans les liaisons ioniques ci-dessus définies - et des groupes $NH_2$ de l'hémoglobine.

15. Procédé selon la revendication 1, caractérisé en ce que le polymère est obtenu à partir de dextrane aminé, de masse moléculaire d'environ 10 000, et de $4.10^{-4}$ moles de butane tetracarboxylique par g de polymère,

– les liaisons ioniques étant établies par l'intermédiaire des groupes carboxylates du butanetricarboxylate fixé sur le polymère, non engagés dans la liaison entre le polymère et l'acide butanetétracarboxylique

– et les liaisons covalentes étant établies entre les autres groupes carboxyliques de l'acide butanetricarboxylique - à la fois non engagés dans la liaison entre l'acide butanetétracarboxylique et le polymère et non engagés dans les liaisons ioniques ci-dessus définies - et des groupes $NH_2$ de l'hémoglobine.

16. Procédé selon la revendication 1, caractérisé en ce que le polymère est obtenu à partir de monométhoxypolyoyéthylène aminé, de masse moléculaire d'environ 5 000, et de $1.5x10^{-4}$ moles de bezenehexacarboxylique par g de polymère,

– les liaisons ioniques étant établies par l'intermédiaire des groupes carboxylates du benzenepentacarboxylate fixé sur le polymère, non engagés dans la liaison entre le polymère et l'acide benzenehexacarboxylique

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

17. Procédé selon la revendication 1, caractérisé en ce que le polymère est obtenu à partir de dextrane, de masse moléculaire d'environ 10 000, et de $1.15x10^{-3}$ moles de benzène-1,2,4,5-tétracarboxylique par g de polymère,

– les liaisons ioniques étant établies par l'intermédiaire des groupes carboxylates du benzènetricarboxylate fixé sur le polymère, non engagés dans la liaison entre le polymère et l'acide benzène-1,2,4,5-tetracarboxylique

– et les liaisons covalentes étant établies entre les autres groupes carboxyliques de l'acide benzènetricarboxylique - à la fois non engagés dans la liaison entre l'acide benzène-1,2,4,5-tétracarboxylique et le polymère et non engagés dans les liaisons ioniques ci-dessus définies - et des groupes $NH_2$ de l'hémoglobine.

18. Procédé selon les revendications 1 à 17, caractérisé en ce que à l'issue de la première étape, le polymère comportant les sites Z est activé avant d'être mis en réaction avec l'hémoglobine.

19. Procédé selon les revendications 1 à 18, caractérisé en ce que l'activation consiste à transformer les fonctions OH en groupes aldéhydes, par exemple par oxydation périodique.

20. Procédé selon les revendications 1 à 19, caractérisé en ce que l'activation des sites Z du polymère et sa mise en réaction avec l'hémoglobine sont pratiquement simultanées.

21. Procédé selon les revendications 1 à 20, caractérisé en ce que les sites Z du polymère :

– sont activés par exemple à l'aide de carbonyldiimidazole, lorsque les sites Z ne comportent pas de groupes aldéhydes, mais qu'ils contiennent des fonctions hydroxyles,

– ou bien sont activés à l'aide de réactifs utilisés en synthèse peptidique, lorsque les sites Z comportent des groupes carboxyles.

22. Procédé selon les revendications 1 à 21, caractérisé en ce que la réaction entre le polymère et l'hémoglobine pendant un temps inférieur à celui entraînant au plus environ 5 % de méthémoglobine, de préférence pendant au plus 10 h, et à une température permettant une conservation correcte de l'hémoglobine, par exemple entre 3°C et 30°C.

23. Procédé de préparation selon l'une des revendications 1 à 22 de conjugués d'hémoglobine dans lesquels les sites Z sont reliés au polymère par l'intermédiaire d'une liaison ester, caractérisé en ce que :

– dans une première étape, on fait réagir les sites Z sous forme anhydride, notamment du benzène 1,2,4,5-tétracarboxylique-dianhydride ou du benzène 1,2,4-tricarboxylique-anhydride, sur un polymère comportant des fonctions OH, notamment du dextrane ou du polyéthylèneglycol, dans un milieu dans lequel le polymère est soluble, pour fixer les sites Z sur le polymère;

– dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris de 5 à 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine.

24. Conjugué macromoléculaire caractérisé en ce qu'il est obtenu par le procédé selon la revendication 3.

25. Conjugué macromoléculaire hydrosoluble d'hémoglobine, non biodégradable ou peu biodégradable, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce qu'il est constitué :

– d'une part par de l'hémoglobine, laquelle peut passer de façon réversible de la forme déoxygénée à la forme oxygénée,

– d'autre part par un polymère P hydrosoluble, non toxique, de préférence non antigénique, hémocompatible, de masse moléculaire d'environ 1 000 à environ 500 000, de préférence d'environ 1 000 à environ 100 000, comportant des groupes polaires, de préférence des groupes hydroxyles, carboxyliques ou amines,

– lequel polymère comporte un ou des sites Z contenant d'une part au moins une charge négative portée par au moins un groupe choisi parmi les groupes suivants: sulfate, phosphate, carboxylate, et destinée à créer une liaison ionique avec le polymère, et contenant d'autre part au moins un groupe carboxylique, aldéhyde ou OH, destiné à créer une liaison covalente avec l'hémoglobine,

– le polymère P étant relié à l'hémoglobine

. d'une part par l'intermédiaire d'au moins une liaison ionique établie entre l'une au moins des charges négatives des sites Z portés par le polymère P et l'hémoglobine, et

. d'autre part par l'intermédiaire d'au moins une liaison covalente établie, entre l'un au moins des groupes carboxylique, aldéhyde ou OH des susdits sites Z portés par le polymère P et l'hémoglobine,

– le nombre de liaisons covalentes entre le polymère et l'hémoglobine étant tel que le conjugué macromoléculaire a une masse moléculaire moyenne d'environ 70 000 à environ 1 000 000, de préférence d'environ 70 000 à environ 500 000, la relation entre le nombre et la nature des charges négatives, le nombre de sites et le nombre de monomères étant la suivante :

– lorsqu'il s'agit de site 2 contenant un groupe anionique unique constitué d'un sulfate ou d'un phosphate, il y a au moins un tel site 2 par chaîne de polymère et au plus un site tous les onze monomères,

– lorsqu'il s'agit de site 2 contenant un groupe anionique unique constitué d'un carboxylate, non engagé dans la liaison entre le site et le polymère et non engagé dans la liaison covalente entre le polymère et l'hémoglobine, il y au moins un tel site 2 par channe de polymère,

– lorsqu'il s'agit de site Z contenant deux charges anioniques provenant de deux carboxylates, non engagés dans la liaison entre le site et le polymère et non engagés dans la liaison covalente entre le polymère et l'hémoglobine, il y a au moins un tel site Z par chaîne de polymère et au plus un site Z tous les six monomères.

26. Conjugué macromoléculaire selon la revendication 25, caractérisé en ce que les liaisons covalentes entre le polymère P et l'hémoglobine sont établies entre au moins un groupe carboxylique ou aldéhyde ou OH porte par les sites 2 et au moins une amine de l'hémoglobine située dans le site allostérique de l'hémoglobine, notamment l'amine de l'une au moins des deux valines β-terminales de l'hémoglobine.

27. Conjugué macromoléculaire selon les revendications 25 et 26, caractérisé en ce que les ponts salins entre les groupes $NH_3^+$ et les groupes $COO^-$ internes de l'hémoglobine sont intacts lorsque l'hémoglobine est sous forme déoxygénée.

28. Conjugué macromoléculaire selon les revendications 26 et 27, caractérisé en ce que le polymère P

est choisi parmi les polysaccharides, notamment les hydroxyalkylamidons dont la radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkylène glycols dans lesquels le groupe alkylène comporte de 2 à 5 atomes de carbone, notamment le polyéthylène glycol et le polypropylène glycol.

29. Conjugué macromoléculaire selon les revendications 25 à 28, caractérisé en ce que le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

30. Conjugué macromoléculaire selon les revendications 25 à 29, caractérisé en ce que le site Z est relié au polymère par l'intermédiaire d'une fonction ester, éther, amide ou amine.

31. Conjugué macromoléculaire selon les revendications 25 à 30, caractérisé en ce que le site Z comporte des groupes $OSO_3H$, $OPO_3H_2$, $O\text{-}CH(COOH)_2$, $\text{-}O\text{-}CH(COOH)\text{-}CH_2\text{-}COOH$,

$$O-CH_2-\left[\left(\underset{\displaystyle COOH}{CH}-CH_2\right)\right]_n-CH_2-COOH,$$

n variant de 1 à environ 4, ou provient du pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3-diphosphoglycérate.

32. Conjugué macromoléculaire selon les revendications 25 à 31, caractérisé en ce que les liaisons covalentes entre le polymère et l'hémoglobine sont établies à partir des groupes carboxyliques provenant des sites Z fixés sur le polymère et des groupes $NH_2$ de l'hémoglobine.

33. Conjugué macromoléculaire selon les revendications 25 à 32, caractérisé en ce que les liaisons ioniques entre le polymère P et l'hémoglobine sont établies entre les groupes carboxylates des sites Z et l'hémoglobine.

34. Conjugué macromoléculaire selon la revendication 25 dans lequel le polymère est du dextrane de masse moléculaire d'environ 10 000, les liaisons ioniques étant établies par l'intermédiaire du groupe carboxylate du benzènedicarboxylate fixé sur le polymère, non engagé dans la liaison entre le benzène-1,2,4-tricarboxylique anhydride et le polymère,

– les liaisons covalentes étant établies entre l'autre groupe carboxylique du benzène-dicarboxylate - à la fois non engagé dans la liaison entre le benzène-1,2,4-tricarboxylique anhydride et le polymère et non engagé dans la liaison ionique définie ci-dessus - et des groupes $NH_2$ de l'hémoglobine, le nombre de groupes benzènedicarboxylates étant de 1 tous les dix monomères.

## Revendications pour l'Etat contractant suivant : ES

1. Procédé de préparation de conjugués macromoléculaires hydrosolubles d'hémoglobine, non biodégradables ou peu biodégradables, pendant le temps durant lequel le conjugué macromoléculaire doit assurer des fonctions oxyphoriques dans le plasma, présentant pour l'oxygène une affinité inférieure à celle de l'hémoglobine libre, caractérisé en ce que :

– on fixe, dans une première étape, des sites Z sur un polymère P, à raison d'au moins un site Z par chaîne de polymère, le polymère P étant hydrosoluble, non toxique, de préférence non antigénique, hémocompatible, de masse moléculaire d'environ 1 000 à environ 500 000, de préférence d'environ 1 000 à environ 100 000, comportant un ou plusieurs groupes polaires, de préférence des groupes hydroxyles, carboxyliques ou amines, et les sites Z contenant d'une part au moins une charge négative portée par des groupes, sulfates et/ou phosphates et/ou carboxylates et destinée à créer une liaison ionique avec l'hémoglobine, et contenant d'autre part au moins un groupe carboxylique, aldéhyde ou OH, destiné à créer une liaison covalente avec l'hémoglobine,

. soit en utilisant un composé Z-Y dans lequel Y est une fonction active ou activable à l'aide d'un agent d'activation, telle que aldéhyde, carboxylique, amine, hydroxyle ou halogène, ou bien

. soit en effectuant un greffage radicalaire des sites Z sur le polymère P ;

– puis dans une seconde étape, on fait réagir le polymère P comportant le ou les sites Z avec de l'hémoglobine sous forme oxygénée dans un milieu non déoxygéné, dans des conditions telles que l'hémoglobine

ne subisse pas de dénaturation et puisse passer après couplage avec le polymère de façon réversible de la forme oxygénée à la forme déoxygénée, en milieu aqueux de pH compris d'environ 5 à environ 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine,

– lorsque la réaction ci-dessus indiquée conduit éventuellement à des fonctions imines, celles-ci peuvent être stabilisées en fonctions amines, par exemple par réduction à l'aide de $NaBH_4$, $NaCNBH_3$, le diméthylaminoborane ou HCOOH.

2. Procédé de préparation de conjugués macromoléculaires selon la revendication 1, caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et l'hémoglobine) soit la suivante :

– lorsque chaque site Z contient un groupe anionique unique constitué d'un sulfate ou d'un phosphate, il y a au moins un tel site Z tous les dix monomères du polymère,

– lorsqu'il s'agit de site Z contenant au moins deux groupes anioniques, constitués de sulfates et/ou de phosphates, il y a au moins un tel site Z par chaîne de polymère,

– lorsque chaque site Z contient des charges anioniques provenant de carboxylates, il faut au moins deux groupes carboxylates sur un même site Z, et au moins un tel site Z tous les cinq monomères,

– lorsque l'un au moins des sites Z contient au moins trois charges négatives provenant de carboxylates, il y a au moins un tel site par chaîne de polymère.

3. Procédé de préparation de conjugués macromoléculaires selon la revendication 1, caractérisé en ce que l'on fixe les sites Z sur le polymère P de telle sorte que la relation entre le nombre et la nature des charges négatives destinés à créer une liaison ionique entre un site et l'hémoglobine (non engagées dans la liaison entre un site et le polymère et non engagées dans la liaison covalente entre un site et l'hémoglobine) soit la suivante :

– lorsqu'il s'agit de site Z contenant un groupe anionique unique constitué d'un sulfate ou d'un phosphate, il y a au moins un tel site Z par chaîne de polymère et au plus un site Z tous les onze monomères,

– lorsqu'il s'agit de site Z contenant un groupe anionique unique constitué d'un carboxylate, non engagé dans la liaison entre le site et le polymère et non engagé dans la liaison covalente entre le polymère et l'hémoglobine, il y au moins un tel site Z par chaîne de polymère,

– lorsqu'il s'agit de site Z contenant deux charges anioniques provenant de deux carboxylates non engagés dans la liaison entre le site et le polymère et non engagé dans la liaison covalente entre le polymère et l'hémoglobine, il y a au moins un site Z par chaîne de polymère et au plus un tel site Z tous les six monomères.

4. Procédé selon les revendications 1 à 3, caractérisé en ce que les liaisons covalentes entre le polymère P et l'hémoglobine sont établies entre au moins un groupe carboxylique, aldéhyde ou OH porté par les sites Z et au moins une amine de l'hémoglobine située dans le site allostérique de l'hémoglobine lorsque celle-ci est sous forme déoxygénée, notamment l'amine de l'une au moins des deux valines β-terminales de l'hémoglobine.

5. Procédé selon les revendications 1 à 4, caractérisé en ce que les ponts salins entre les groupes $NH_3^+$ et les groupes $COO^-$ internes de l'hémoglobine sont intacts lorsque l'hémoglobine est sous forme déoxygénée.

6. Procédé selon les revendirations 1 à 5, caractérisé en ce que le polymère P est choisi parmi les polysaccharides, notamment les hydroxyalkylamidons dont la radical alkyle comporte de 2 à 4 atomes de carbone, l'inuline, le dextrane et ses dérivés, notamment le dextrane aminé, l'alcool polyvinylique, la polyvinylpyrrolidone, le polyméthacrylate et ses dérivés, les polypeptides, les polyalkylène glycols dans lesquels le groupe alkylène comporte de 2 à 5 atomes de carbone, notamment le polyéthylène glycol et le polypropylène glycol.

7. Procédé selon les revendications 1 à 6, caractérisé en ce que le polymère P a une masse moléculaire moyenne inférieure ou égale à 70 000, lorsqu'il est constitué par le dextrane et ses dérivés, et une masse moléculaire inférieure ou égale à 10 000 lorsqu'il est choisi parmi les polyalkylène glycols, la polyvinylpyrrolidone ou le polyméthylacrylate.

8. Procédé selon les revendications 1 à 7, caractérisé en ce que le site Z est relié au polymère par l'intermédiaire d'une fonction ester, éther, amide ou amine.

9. Procédé selon les revendications 1 à 8, caractérisé en ce que le site Z comporte les groupes $OSO_3H$, $OPO_3H_2$, $O\text{-}CH(COOH)_2$, $\text{-}O\text{-}CH(COOH)\text{-}CH_2\text{-}COOH$,

$$O-CH_2-\left[\begin{matrix}(\underset{|}{CH}-CH_2) \\ COOH\end{matrix}\right]_n-CH_2-COOH,$$

n variant de 1 à environ 4, ou provient du pyridoxalsulfate, du pyridoxalphosphate, de l'adénosine triphosphate, de la phosphotyrosine, de la phosphosérine, de l'inositolhéxaphosphate et ses dérivés, d'acide polycarboxylique contenant de 2 à 10 atomes de carbone sur la chaîne principale, de l'acide benzènecarboxylique comportant au moins trois fonctions carboxyliques, du 2,3-diphosphoglycérate.

10. Procédé selon les revendications 1 à 9, caractérisé en ce que les liaisons covalentes entre le polymère et l'hémoglobine sont établies à partir des groupes carboxyliquees provenant des sites Z fixés sur le polymère et des groupes $NH_2$ de l'hémoglobine.

11. Procédé selon les revendications 1 à 10, caractérisé en ce que les liaisons ioniques entre le polymère P et l'hémoglobine sont établies entre les groupes carboxylates des sites Z et l'hémoglobine.

12. Procédé selon la revendication 1, caractérisé en ce que le polymère est obtenu à partir de dextrane aminé de masse moléculaire d'environ 40 000, et de $2.10^{-4}$ moles d'acide benzenehexacarboxylique par g de dextrane,

– les liaisons ioniques étant établies par l'intermédiaire des groupes carboxylates du benzenepentacarboxylate fixé sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

13. Procédé selon la revendication 1, caractérisé en ce que le polymère est obtenu à partir du dextrane aminé, de masse moléculaire d'environ 10 000, et de $3.5x10^{-4}$ moles d'acide benzenehexacarboxylique par g de dextrane,

– les liaisons ioniques étant établies par l'intermédiaire des groupes carboxylates du benzenepentacarboxylate fixés sur le polymère, non engagés dans la liaison entre le polymère et l'acide benzenehexacarboxylique,

– et les liaisons covalentes étant établies entre les autres groupes carboxyliques de l'acide benzenepentacarboxylique - à la fois non engagés dans la liaison entre l'acide benzenehexacarboxylique et le polymère et non engagés dans les liaisons ioniques ci-dessus définies - et des groupes $NH_2$ de l'hémoglobine.

14. Procédé selon la revendication 1, caractérisé en ce que le polymère est obtenu à partir de dextrane aminé, de masse moléculaire d'environ 10 000, et de $3,2x10^{-4}$ moles de benzenetetracarboxylique par g de dextrane,

– les liaisons ioniques étant établies par l'intermédiaire des groupes carboxylates du benzenetricarboxylate fixé sur le polymère, non engagés dans la liaison entre le polymère et l'acide benzenetetracarboxylique,

– et les liaisons covalentes étant établies entre les autres groupes carboxyliques de l'acide benzenetricarboxylique - à la fois non engagés dans la liaison entre l'acide benzenetetracarboxylique et le polymère et non engagés dans les liaisons ioniques ci-dessus définies - et des groupes $NH_2$ de l'hémoglobine.

15. Procédé selon la revendication 1, caractérisé en ce que le polymère est obtenu à partir de dextrane aminé, de masse moléculaire d'environ 10 000, et de $4.10^{-4}$ moles de butane tetracarboxylique par g de polymère,

– l'intermédiaire des groupes carboxylates du butanetricarboxylate fixé sur le polymère, non engagés dans la liaison entre le polymère et l'acide butanetétracarboxylique

– et les liaisons covalentes étant établies entre les autres groupes carboxyliques de l'acide butanetricarboxylique - à la fois non engagés dans la liaison entre l'acide butanetétracarboxylique et le polymère et non engagés dans les liaisons ioniques ci-dessus définies - et des groupes $NH_2$ de l'hémoglobine.

16. Procédé selon la revendication 1, caractérisé en ce que le polymère est obtenu à partir de monométhoxypolyoyéthylène aminé, de masse moléculaire d'environ 5 000, et de $1.5x10^{-4}$ moles de bezenehexacarboxylique par g de polymère,

– les liaisons ioniques étant établies par l'intermédiaire des groupes carboxylates du benzenepentacarboxylate fixé sur le polymère, non engagés dans la liaison entre le polymère et l'acide benzenehexacarboxylique

– et les liaisons covalentes étant établies entre les autres groupes carboxyliques de l'acide benzenepentacarboxylique - à la fois non engagés dans la liaison entre l'acide benzenehexacarboxylique et le polymère

et non engagés dans les liaisons ioniques ci-dessus définies - et des groupes $NH_2$ de l'hémoglobine.

17. Procédé selon la revendication 1, caractérisé en ce que le polymère est obtenu à partir de dextrane, de masse moléculaire d'environ 10 000, et de $1.15x10^{-3}$ moles de benzène-1,2,4,5-tétracarboxylique par g de polymère,

– les liaisons ioniques étant établies par l'intermédiaire des groupes carboxylates du benzènetricarboxylate fixé sur le polymère, non engagés dans la liaison entre le polymère et l'acide benzène-1,2,4,5-tétracarboxylique

– et les liaisons covalentes étant établies entre les autres groupes carboxyliques de l'acide benzènetricarboxylique - à la fois non engagés dans la liaison entre l'acide benzène-1,2,4,5-tétracarboxylique et le polymère et non engagés dans les liaisons ioniques ci-dessus définies - et des groupes $NH_2$ de l'hémoglobine.

18. Procédé selon les revendications 1 à 17, caractérisé en ce que à l'issue de la première étape, le polymère comportant les sites Z est activé avant d'être mis en réaction avec l'hémoglobine.

19. Procédé selon les revendications 1 à 18, caractérisé en ce que l'activation consiste à transformer les fonctions OH en groupes aldéhydes, par exemple par oxydation périodique.

20. procédé selon les revendications 1 à 19, caractérisé en ce que l'activation des sites Z du polymère et sa mise en réaction avec l'hémoglobine sont pratiquement simultanées.

21. Procédé selon les revendications 1 à 20, caractérisé en ce que les sites Z du polymère :

– sont activés par exemple à l'aide de carbonyldiimidazole, lorsque les sites Z ne comportent pas de groupes aldéhydes, mais qu'ils contiennent des fonctions hydroxyles,

– ou bien sont activés à l'aide de réactifs utilisés en synthèse peptidique, lorsque les sites Z comportent des groupes carboxyles.

22. Procédé selon les revendications 1 à 21, caractérisé en ce que la réaction entre le polymère et l'hémoglobine pendant un temps inférieur à celui entraînant au plus environ 5 % de méthémoglobine, de préférence pendant au plus 10 h, et à une température permettant une conservation correcte de l'hémoglobine, par exemple entre 3°C et 30°C.

23. procédé de préparation selon l'une des revendications 1 à 22 de conjugués d'hémoglobine dans lesquels les sites Z sont reliés au polymère par l'intermédiaire d'une liaison ester, caractérisé en ce que :

– dans une première étape, on fait réagir les sites Z sous forme anhydride, notamment du benzène 1,2,4,5-tétracarboxylique-dianhydride ou du benzène 1,2,4-tricarboxylique-anhydride, sur un polymère comportant des fonctions OH, notamment du dextrane ou du polyéthylèneglycol, dans un milieu dans lequel le polymère est soluble, pour fixer les sites Z sur le polymère;

– dans une seconde étape, on fait réagir le polymère P comportant le ou les sites Z avec de l'hémoglobine sous forme oxygénée, dans un milieu non déoxygéné, dans des conditions telles que l'hémoglobine ne subisse pas de dénaturation et puisse passer après couplage avec le polymère de façon réversible de la forme oxygénée à la forme déoxygénée, en milieu aqueux de pH compris de 5 à 9,

pour former d'une part au moins une liaison ionique entre l'un au moins des sites Z portés par le polymère et l'hémoglobine et d'autre part au moins une liaison covalente entre le même susdit site Z porté par le polymère et l'hémoglobine.

## Claims

### Claims for the following Contracting States : AT, BE, CH, DE, FR, GB, GR, IT, LI, LU, NL, SE

1. Procedure for the preparation of water-soluble macromolecular conjugates of hemoglobin, only slightly or not at all biodegradable during the period of time during which the macromolecular conjugate must exercise its oxygen-carrying functions in the plasma, exhibiting an affinity for oxygen less than that of free hemoglobin, characterized in that:

– in the first step, Z sites are bound to a polymer P, to the extent of at least one Z site per polymer chain, the polymer P being water-soluble, non-toxic, preferably non-antigenic, hemocompatible, of molecular mass of about 1,000 to about 500,000, and preferably of about 1,000 to about 100,000, containing one or more polar groups, preferably hydroxyl, carboxylic or amine groups, and the Z sites containing, on the one hand, at least one negative charge borne by sulfate and/or phosphate and/or carboxylate groups and intended to lead to the formation of an ionic bond with hemoglobin and containing, on the other hand, at least one carboxylic, aldehyde or OH group intended to lead to the formation of a covalent bond with hemoglobin,

. either by using a compound Z-Y in which Y is an active function or can be activated using an activating agent, such as aldehyde, carboxylic, amine, hydroxyl or halogen,

. or by carrying out a radical reaction at the Z sites on the polymer P;

– then in the second step, the polymer P containing the Z site(s) is made to react with hemoglobin in the oxygenated form in a non-deoxygenated medium under conditions such that the hemoglobin does not undergo denaturation and can pass, after coupling to the polymer, in a reversible manner from the oxygenated form to the deoxygenated form in aqueous medium at a pH varying between about 5 and about 9,

in order to form, on the one hand, at least one ionic linkage between at least one of the Z sites borne by the polymer and hemoglobin and, on the other, at least one covalent linkage between the same Z site mentioned above borne by the polymer and hemoglobin,

– when the above-mentioned reaction may lead to imine functions, these latter may be stabilized as amine functions by reduction, for example, using $NaBH_4$, $NaCNBH_3$, dimethylaminoborane or HCOOH.

2. Procedure for the preparation of macromolecular conjugates according to Claim 1, characterized in that the Z sites are bound to the polymer P such that the relationship between the number and the nature of the negative charges destined to lead to the formation of an ionic bond between a site and hemoglobin (charges not involved in linkage between a site and a polymer and not involved in covalent linkage between a site and hemoglobin) is the following:

– when each Z site contains a unique anionic group constituted by a sulfate or a phosphate, there is at least one such Z site for every ten monomers of the polymer,

– when a Z site contains at least two anionic groups constituted by sulfates and/or phosphates, there is at least one such Z site per polymer chain,

– when each Z site contains anionic charges derived from carboxylates, there must be at least two carboxylate groups at the same Z site, and at least one such Z site for every five monomers,

– when at least one of the Z sites contains at least three negative charges derived from carboxylates, there is at least one such site per polymer chain.

3. Procedure for the preparation of macromolecular conjugates according to Claim 1, characterized in that the Z sites are bound to the polymer P such that the relationship between the number and the nature of the negative charges intended to lead to the formation of an ionic linkage between a site and hemoglobin (charges not involved in linkage between a site and the polymer and not involved in covalent linkage between a site and hemoglobin) is the following:

– when a Z site contains a unique anionic group constituted by a sulfate or a phosphate, there is at least one such Z site per polymer chain and not more than one Z site for every eleven monomers,

– when a Z site contains a unique anionic group constituted by a carboxylate not involved in linkage between the site and the polymer and not involved in covalent linkage between the polymer and hemoglobin, there is at least one such Z site per polymer chain,

– when a Z site contains two anionic charges derived from two carboxylates not involved in linkage between the site and the polymer and not involved in covalent linkage between the polymer and hemoglobin, there is at least one Z site per polymer chain and not more than one such Z site for every six monomers.

4. Procedure according to the Claims 1 to 3, characterized in that the covalent linkages between the polymer P and hemoglobin are established between at least a carboxylic, aldehyde or OH group borne by the Z sites and at least one amino group of hemoglobin located at the allosteric site of hemoglobin when this latter is in the deoxygenated form, in particular the amino group of at least one of the two β-terminal valines of hemoglobin.

5. Procedure according to the Claims 1 to 4, characterized in that the salt bridges between the $NH_3^+$ groups and the internal $COO^-$ groups of the hemoglobin molecule are intact when hemoglobin is in the deoxygenated form.

6. Procedure according to the Claims 1 to 5, characterized in that the polymer P is selected from among the polysaccharides, in particular the hydroxyalkyl starches in which the alkyl radical contains from 2 to 4 carbon atoms, inulin, dextran and its derivatives, in particular aminated dextran, polyvinyl alcohol, polyvinylpyrrolidone, polymethacrylate and its derivatives, polypeptides, polyalkylene glycols in which the alkylene group contains from 2 to 5 carbon atoms, in particular polyethylene glycol and polypropylene glycol.

7. Procedure according to the Claims 1 to 6, characterized in that the polymer P has an average molecular mass lower than or equal to 70,000, when it is constituted by dextran and its derivatives, and a molecular mass lower than or equal to 10,000 when it is chosen from among the polyalkylene glycols, polyvinylpyrrolidone or polymethylacrylate.

8. Procedure according to the Claims 1 to 7, characterized in that the Z site is linked to the polymer by the intermediary of an ester, ether, amide or amine function.

9. Procedure according to the Claims 1 to 8, characterized in that the Z site contains the groups $OSO_3H$, $OPO_3H_2$, $O\text{-}CH(COOH)_2$, $\text{-}O\text{-}CH(COOH)\text{-}CH_2\text{-}COOH$,

$$O-CH_2-\left[\begin{matrix}(CH-CH_2)\\ | \\ COOH\end{matrix}\right]_n-CH_2-COOH,$$

n varying from 1 to about 4, or is derived from pyridoxalsulfate, pyridoxalphosphate, adenosine triphosphate, phosphotyrosine, phosphoserine, inositol hexaphosphate and its derivatives, polycarboxylic acids containing from 2 to 10 carbon atoms in the main chain, benzene carboxylic acids containing at least three carboxylic functions, 2,3-diphosphoglycerate.

10. Procedure according to the Claims 1 to 9, characterized in that the covalent linkages between the polymer and hemoglobin are established through the carboxylic groups present at the Z sites bound to the polymer and $NH_2$ groups of hemoglobin.

11. Procedure according to the Claims 1 to 10, characterized in that the ionic linkages between the polymer P and hemoglobin are established between the carboxylate groups of the Z sites and hemoglobin.

12. Procedure according to Claim 1, characterized in that the polymer is obtained starting from aminated dextran of molecular mass of about 40,000, and $2x10^{-4}$ moles of benzene hexacarboxylic acid per g of dextran,

– the ionic linkages being established by the intermediary of carboxylate groups of the benzene pentacarboxylate bound to the polymer and not involved in linkage between the polymer and the benzene hexacarboxylic acid,

– and the covalent linkages being established between the other carboxylic groups of the benzene pentacarboxylic acid - implicated in neither linkage between the benzene hexacarboxylic acid and the polymer nor in the ionic linkages specified above - and $NH_2$ groups of hemoglobin.

13. Procedure according to Claim 1, characterized in that the polymer is obtained starting from aminated dextran of molecular mass of about 10,000 and $3.5x10^{-4}$ moles of benzene hexacarboxylic acid per g of dextran,

– the ionic linkages being established by the intermediary of carboxylate groups of the benzene pentacarboxylate bound to the polymer and not involved in linkage between the polymer and the benzene hexacarboxylic acid,

– and the covalent linkages being established between the other carboxylic groups of the benzene pentacarboxylic acidimplicated in neither linkage between the benzene hexacarboxylic acid and the polymer nor in the ionic linkages specified above - and $NH_2$ groups of hemoglobin.

14. Procedure according to Claim 1, characterized in that the polymer is obtained starting from aminated dextran of molecular mass of about 10,000, and $3.2x10^{-4}$ moles of benzene tetracarboxylic acid per g of dextran,

– the ionic linkages being established by the intermediary of carboxylate groups of the benzene tricarboxylate bound to the polymer and not involved in linkage between the polymer and the benzene tetracarboxylic acid,

– and the covalent linkages being established between the other carboxylic groups of the benzene tricarboxvlic acid - implicated in neither linkage between the benzene tetracarboxylic acid and the polymer nor in the ionic linkages specified above - and $NH_2$ groups of hemoglobin.

15. Procedure according to Claim 1, characterized in that the polymer is obtained starting from aminated dextran of molecular mass of about 10,000, and $4x10^{-4}$ moles of butane tetracarboxylic acid per g of polymer,

– the ionic linkages being established by the intermediary of the carboxylate groups of the butane tricarboxylate bound to the polymer and not involved in linkage between the polymer and the butane tetracarboxylic acid,

– and the covalent linkages being established between the other carboxylic groups of the butane tricarboxylic acid - implicated in neither linkage between the butane tetracarboxylic acid and the polymer nor in the ionic linkages specified above - and $NH_2$ groups of hemoglobin.

16. Procedure according to Claim 1, characterized in that the polymer is obtained starting from aminated monomethoxypolyoxyethylene of molecular mass of about 5,000, and $1.5x10^{-4}$ moles of benzene hexacarboxylic acid per g of polymer,

– the ionic linkages being established by the intermediary of the carboxylate groups of the benzene pentacarboxylate bound to the polymer and not involved in linkage between the polymer and the benzene hexacarboxylic acid,

– and the covalent linkages being established between the other carboxylic groups of the benzene pentacarboxylic acid - implicated in neither linkage between the benzene hexacarboxylic acid and the polymer nor in the ionic linkages specified above - and $NH_2$ groups of hemoglobin.

17. Procedure according to Claim 1, characterized in that the polymer is obtained starting from dextran of molecular mass of about 10,000, and $1.15x10^{-3}$ moles of 1,2,4,5-benzene tetracarboxylic acid per g of polymer,

– the ionic linkages being established by the intermediary of the carboxylate groups of the benzene tricarboxylate bound to the polymer and not involved in linkage between the polymer and the 1,2,4,5-benzene

tetracarboxylic acid,

– and the covalent linkages being established between the other carboxylic groups of the benzene tricarboxylic acid - implicated in neither linkage between the 1,2,4,5-benzene tetracarboxylic acid and the polymer nor in the ionic linkages specified above - and $NH_2$ groups of hemoglobin.

18. Procedure according to the Claims 1 to 17, characterized in that at the end of the first step, the polymer containing the Z sites is activated before being made to react with hemoglobin.

19. Procedure according to the Claims 1 to 18, characterized in that the activation consists of converting the OH functions to aldehyde groups by periodate oxidation, for example.

20. Procedure according to the Claims 1 to 19, characterized in that the activation of the Z sites of the polymer and its reaction with hemoglobin are practically simultaneous.

21. Procedure according to the Claims 1 to 20, characterized in that the Z sites of the polymer:

– are activated, for example, using carbonyl diimidazole, when the Z sites do not contain aldehyde groups but when they contain hydroxyl functions,

– or are activated using reagents used in peptide synthesis when the Z sites contain carboxyl groups.

22. Procedure according to the Claims 1 to 21, characterized in that the reaction between the polymer and hemoglobin is carried out for a time shorter than that leading to the formation of more than about 5% of methemoglobin, and preferably not exceeding 10 h, and at a temperature at which the hemoglobin is not denatured, for example, between 3°C and 30°C.

23. Procedure for the preparation according to any one of the Claims 1 to 22 of hemoglobin conjugates in which the Z sites are linked to the polymer by the intermediary of an ester linkage, characterized in that:

– in the first step, the Z sites are made to react in the form of an anhydride, in particular of 1,2,4,5-benzene tetracarboxylic acid dianhydride or of 1,2,4-benzene tricarboxylic acid anhydride, with a polymer containing OH functions, in particular dextran or polyethylene glycol, in a medium in which the polymer is soluble in order to bind the Z sites to the polymer;

– in the second step, the polymer P containing the Z site(s) is made to react with hemoglobin in the oxygenated form in a non-deoxygenated medium under conditions such that the hemoglobin does not undergo denaturation and can pass, after coupling to the polymer, in a reversible manner from the oxygenated form to the deoxygenated form in an aqueous medium at a pH varying between 5 and 9,

in order to form, on the one hand, at least one ionic linkage between at least one of the Z sites borne by the polymer and hemoglobin and, on the other, at least one covalent linkage between the same Z site mentioned above borne by the polymer and hemoglobin.

24. Macromolecular conjugate characterized in that it is obtained by the procedure according to Claim 3.

25. Water-soluble macromolecular conjugate of hemoglobin, only slightly or not at all biodegradable during the period of time during which the macromolecular conjugate must exercise its oxygen-carrying functions in the plasma, exhibiting an affinity for oxygen less than that of free hemoglobin, characterized in that it is constituted:

– on the one hand, by hemoglobin, which can pass in a reversible manner from the deoxygenated form to the oxygenated form,

– on the other hand, by a water-soluble polymer P, non-toxic, preferably non-antigenic, hemocompatible, of molecular mass of about 1,000 to about 500,000, and preferably of about 1,000 to about 100,000, containing polar groups, preferably hydroxyl, carboxylic or amine groups,

– this polymer contains one or more Z sites containing, on the one hand, at least one negative charge borne by at least one group chosen from among the following groups: sulfate, phosphate, carboxylate, and intended to lead to the formation of an ionic linkage to the polymer and containing, on the other hand, at least one carboxylic, aldehyde or OH group, intended to lead to the formation of a covalent linkage to hemoglobin,

– the polymer P being linked to hemoglobin

. on the one hand, by the intermediary of at least one ionic linkage established between at least one of the negative charges of the Z sites borne by the polymer P and hemoglobin, and

. on the other hand, by the intermediary of at least one covalent linkage established between at least one of the carboxylic, aldehyde or OH groups of the above-mentioned Z sites borne by the polymer P and hemoglobin,

– the number of covalent linkages between the polymer and hemoglobin being such that the macromolecular conjugate has an average molecular mass of from about 70,000 to about 1,000,000, and preferably from about 70,000 to about 500,000, the relationship between the number and the nature of the negative charges, the number of sites and the number of monomers being the following:

– when a Z site contains a unique anionic group constituted by a sulfate or a phosphate, there is at least one such Z site per polymer chain and not more than one site for every eleven monomers,

– when the Z site contains a unique anionic group constituted by a carboxylate, not involved in linkage between the site and the polymer and not involved in covalent linkage between the polymer and hemoglobin, there is at least one such Z site per polymer chain,

– when a Z site contains two anionic charges derived from two carboxylates, not involved in linkage between the site and the polymer and not involved in covalent linkage betweeen the polymer and hemoglobin, there is at least one such Z site per polymer chain and not more than one Z site for every six monomers.

26. Macromolecular conjugate according to Claim 25, characterized in that the covalent linkages between the polymer P and hemoglobin are established between at least one carboxylic or aldehyde or OH group borne by the Z sites and at least one amino group of hemoglobin located at the allosteric site of hemoglobin, in particular the amino group of at least one of the two β-terminal valines of hemoglobin.

27. Macromolecular conjugate according to the Claims 25 and 26, characterized in that the salt bridges between the $NH_3^+$ groups and the internal $COO^-$ of the hemoglobin molecule are intact when the hemoglobin is in the deoxygenated form.

28. Macromolecular conjugate according to the Claims 26 and 27, characterized in that the polymer P is selected from among the polysaccharides, in particular the hydroxyalkyl starches in which the alkyl radical contains from 2 to 4 carbon atoms, inulin, dextran and its derivatives, in particular aminated dextran, polyvinyl alcohol, polyvinylpyrrolidone, polymethacrylate and its derivatives, polypeptides, polyalkylene glycols in which the alkylene group contains from 2 to 5 carbon atoms, in particular polyethylene glycol and polypropylene glycol.

29. Macromolecular conjugate according to the Claims 25 to 28, characterized in that the polymer P has an average molecular mass lower than or equal to 70,000 when it is constituted by dextran and its derivatives, and a molecular mass lower than or equal to 10,000 when it is chosen from among the polyalkylene glycols, polyvinylpyrrolidone or polymethylacrylate.

30. Macromolecular conjugate according to the Claims 25 to 29, characterized in that the Z site is linked to the polymer by the intermediary of an ester, ether, amide or amine function.

31. Macromolecular conjugate according to the Claims 25 to 30, characterized in that the Z site contains the groups $OSO_3H$, $OPO_3H_2$, $O\text{-}CH(COOH)_2$, $\text{-}O\text{-}CH(COOH)\text{-}CH_2\text{-}COOH$,

$$O-CH_2-\left[(\underset{\displaystyle COOH}{\underset{|}{CH}}-CH_2)\right]_n-CH_2-COOH,$$

n varying from 1 to about 4, or is derived from pyridoxal sulfate, pyridoxal phosphate, adenosine triphosphate, phosphotyrosine, phosphoserine, inositol hexaphosphate and its derivates, polycarboxylic acids containing from 2 to 10 carbon atoms in the main chain, benzene carboxylic acids containing at least three carboxylic functions, 2,3-diphosphoglycerate.

32. Macromolecular conjugate according to the Claims 25 to 31, characterized in that the covalent linkages between the polymer and hemoglobin are established through carboxylic groups derived from the Z sites bound to the polymer and $NH_2$ groups of hemoglobin.

33. Macromolecular conjugate according to the Claims 25 to 32, characterized in that the ionic linkages between the polymer P and hemoglobin are established between the carboxylate groups of the Z sites and hemoglobin.

34. Macromolecular conjugate according to claim 25, in which the polymer dextran of molecular mass of about 10,000, the ionic linkages being established by the intermediary of the carboxylate group of benzene dicarboxylate bound to the polymer and not involved in linkage between the 1,2,4-benzene tricarboxylic acid anhydride and the polymer,

– the covalent linkages being established between the other carboxylic group of benzene dicarboxylate - implicated in neither linkage between the 1,2,4-benzene tricarboxylic acid anhydride and the polymer nor in the ionic linkage specified above - and $NH_2$ groups of hemoglobin, the number of benzene dicarboxylate groups being 1 for every ten monomers.

**Claims for the following Contracting State : ES**

1. Procedure for the preparation of water-soluble macromolecular conjugates of hemoglobin, only slightly or not at all biodegradable during the period of time during which the macromolecular conjugate must exercise its oxygen-carrying functions in the plasma, exhibiting an affinity for oxygen less than that of free hemoglobin, characterized in that:

– in the first step, Z sites are bound to a polymer P, to the extent of at least one Z site per polymer chain,

the polymer P being water-soluble, non-toxic, preferably non-antigenic, hemocompatible, of molecular mass of about 1,000 to about 500,000, and preferably of about 1,000 to about 100,000, containing one or more polar groups, preferably hydroxyl, carboxylic or amine groups, and the Z sites containing, on the one hand, at least one negative charge borne by sulfate and/or phosphate and/or carboxylate groups and intended to lead to the formation of an ionic bond with hemoglobin and containing, on the other hand, at least one carboxylic, aldehyde or OH group intended to lead to the formation of a covalent bond with hemoglobin,

. either by using a compound Z-Y in which Y is an active function or can be activated using an activating agent, such as aldehyde, carboxylic, amine, hydroxyl or halogen,

. or by carrying out a radical reaction at the Z sites on the polymer P;

– then in the second step, the polymer P containing the Z site(s) is made to react with hemoglobin in the oxygenated form in a non-deoxygenated medium under conditions such that the hemoglobin does not undergo denaturation and can pass, after coupling to the polymer, in a reversible manner from the oxygenated form to the deoxygenated form in aqueous medium at a pH varying between about 5 and about 9,

in order to form, on the one hand, at least one ionic linkage between at least one of the Z sites borne by the polymer and hemoglobin and, on the other, at least one covalent linkage between the same Z site mentioned above borne by the polymer and hemoglobin,

– when the above-mentioned reaction may lead to imine functions, these latter may be stabilized as amine functions by reduction, for example, using $NaBH_4$, $NaCNBH_3$, dimethylaminoborane or HCOOH.

2. Procedure for the preparation of macromolecular conjugates according to Claim 1, characterized in that the Z sites are bound to the polymer P such that the relationship between the number and the nature of the negative charges destined to lead to the formation of an ionic bond between a site and hemoglobin (charges not involved in linkage between a site and a polymer and not involved in covalent linkage between a site and hemoglobin) is the following:

– when each Z site contains a unique anionic group constituted by a sulfate or a phosphate, there is at least one such Z site for every ten monomers of the polymer,

– when a Z site contains at least two anionic groups constituted by sulfates and/or phosphates, there is at least one such Z site per polymer chain,

– when each Z site contains anionic charges derived from carboxylates, there must be at least two carboxylate groups at the same Z site, and at least one such Z site for every five monomers,

– when at least one of the Z sites contains at least three negative charges derived from carboxylates, there is at least one such site per polymer chain.

3. Procedure for the preparation of macromolecular conjugates according to Claim 1, characterized in that the Z sites are bound to the polymer P such that the relationship between the number and the nature of the negative charges intended to lead to the formation of an ionic linkage between a site and hemoglobin (charges not involved in linkage between a site and the polymer and not involved in covalent linkage between a site and hemoglobin) is the following:

– when a Z site contains a unique anionic group constituted by a sulfate or a phosphate, there is at least one such Z site per polymer chain and not more than one Z site for every eleven monomers,

– when a Z site contains a unique anionic group constituted by a carboxylate not involved in linkage between the site and the polymer and not involved in covalent linkage between the polymer and hemoglobin, there is at least one such Z site per polymer chain,

– when a Z site contains two anionic charges derived from two carboxylates not involved in linkage between the site and the polymer and not involved in covalent linkage between the polymer and hemoglobin, there is at least one Z site per polymer chain and not more than one such Z site for every six monomers.

4. Procedure according to the Claims 1 to 3, characterized in that the covalent linkages between the polymer P and hemoglobin are established between at least a carboxylic, aldehyde or OH group borne by the Z sites and at least one amino group of hemoglobin located at the allosteric site of hemoglobin when this latter is in the deoxygenated form, in particular the amino group of at least one of the two β-terminal valines of hemoglobin.

5. Procedure according to the Claims 1 to 4, characterized in that the salt bridges between the $NH_3^+$ groups and the internal $COO^-$ groups of the hemoglobin molecule are intact when hemoglobin is in the deoxygenated form.

6. Procedure according to the Claims 1 to 5,,characterized in that the polymer P is selected from among the polysaccharides, in particular the hydroxyalkyl starches in which the alkyl radical contains from 2 to 4 carbon atoms, inulin, dextran and its derivatives, in particular aminated dextran, polyvinyl alcohol, polyvinylpyrrolidone, polymethacrylate and its derivatives, polypeptides, polyalkylene glycols in which the alkylene group contains from 2 to 5 carbon atoms, in particular polyethylene glycol and polypropylene glycol.

7. Procedure according to the Claims 1 to 6, characterized in that the polymer P has an average molecular

mass lower than or equal to 70,000, when it is constituted by dextran and its derivatives, and a molecular mass lower than or equal to 10,000 when it is chosen from among the polyalkylene glycols, polyvinylpyrrolidone or polymethylacrylate.

8. Procedure according to the Claims 1 to 7, characterized in that the Z site is linked to the polymer by the intermediary of an ester, ether, amide or amine function.

9. Procedure according to the Claims 1 to 8, characterized in that the Z site contains the groups $OSO_3H$, $OPO_3H_2$, $O-CH(COOH)_2$, $-O-CH(COOH)-CH_2-COOH$,

$$O-CH_2-\left[\underset{\displaystyle COOH}{(CH}-CH_2)\right]_n-CH_2-COOH,$$

n varying from 1 to about 4, or is derived from pyridoxalsulfate, pyridoxalphosphate, adenosine triphosphate, phosphotyrosine, phosphoserine, inositol hexaphosphate and its derivatives, polycarboxylic acids containing from 2 to 10 carbon atoms in the main chain, benzene carboxylic acids containing at least three carboxylic functions, 2,3-diphosphoglycerate.

10. Procedure according to the Claims 1 to 9, characterized in that the covalent linkages between the polymer and hemoglobin are established through the carboxylic groups present at the Z sites bound to the polymer and $NH_2$ groups of hemoglobin.

11. Procedure according to the Claims 1 to 10, characterized in that the ionic linkages between the polymer P and hemoglobin are established between the carboxylate groups of the Z sites and hemoglobin.

12. Procedure according to Claim 1, characterized in that the polymer is obtained starting from aminated dextran of molecular mass of about 40,000, and $2x10^{-4}$ moles of benzene hexacarboxylic acid per g of dextran,

– the ionic linkages being established by the intermediary of carboxylate groups of the benzene pentacarboxylate bound to the polymer and not involved in linkage between the polymer and the benzene hexacarboxylic acid,

– and the covalent linkages being established between the other carboxylic groups of the benzene pentacarboxylic acid - implicated in neither linkage between the benzene hexacarboxylic acid and the polymer nor in the ionic linkages specified above - and $NH_2$ groups of hemoglobin.

13. Procedure according to Claim 1, characterized in that the polymer is obtained starting from aminated dextran of molecular mass of about 10,000 and $3.5x10^{-4}$ moles of benzene hexacarboxylic acid per g of dextran,

– the ionic linkages being established by the intermediary of carboxylate groups of the benzene pentacarboxylate bound to the polymer and not involved in linkage between the polymer and the benzene hexacarboxylic acid,

– and the covalent linkages being established between the other carboxylic groups of the benzene pentacarboxylic acid - implicated in neither linkage between the benzene hexacarboxylic acid and the polymer nor in the ionic linkages specified above - and $NH_2$ groups of hemoglobin.

14. Procedure according to Claim 1, characterized in that the polymer is obtained starting from aminated dextran of molecular mass of about 10,000, and $3.2x10^{-4}$ moles of benzene tetracarboxylic acid per g of dextran,

– the ionic linkages being established by the intermediary of carboxylate groups of the benzene tricarboxylate bound to the polymer and not involved in linkage between the polymer and the benzene tetracarboxylic acid,

– and the covalent linkages being established between the other carboxylic groups of the benzene tricarboxylic acid - implicated in neither linkage between the benzene tetracarboxylic acid and the polymer nor in the ionic linkages specified above - and $NH_2$ groups of hemoglobin.

15. Procedure according to Claim 1, characterized in that the polymer is obtained starting from aminated dextran of molecular mass of about 10,000, and $4x10^{-4}$ moles of butane tetracarboxylic acid per g of polymer,

– the ionic linkages being established by the intermediary of the carboxylate groups of the butane tricarboxylate bound to the polymer and not involved in linkage between the polymer and the butane tetracarboxylic acid,

– and the covalent linkages being established between the other carboxylic groups of the butane tricarboxylic acid - implicated in neither linkage between the butane tetracarboxylic acid and the polymer nor in the ionic linkages specified above - and $NH_2$ groups of hemoglobin.

16. Procedure according to Claim 1, characterized in that the polymer is obtained starting from aminated monomethoxypolyoxyethylene of molecular mass of about 5,000, and $1.5x10^{-4}$ moles of benzene hexacarboxylic acid per g of polymer,

– the ionic linkages being established by the intermediary of the carboxylate groups of the benzene pentacarboxylate bound to the polymer and not involved in linkage between the polymer and the benzene

hexacarboxylic acid,

– and the covalent linkages being established between the other carboxylic groups of the benzene pentacarboxylic acid - implicated in neither linkage between the benzene hexacarboxylic acid and the polymer nor in the ionic linkages specified above - and $NH_2$ groups of hemoglobin.

17. Procedure according to Claim 1, characterized in that the polymer is obtained starting from dextran of molecular mass of about 10,000, and $1.15x10^{-3}$ moles of 1,2,4,5-benzene tetracarboxylic acid per g of polymer,

– the ionic linkages being established by the intermediary of the carboxylate groups of the benzene tricarboxylate bound to the polymer and not involved in linkage between the polymer and the 1,2,4,5-benzene tetracarboxylic acid,

– and the covalent linkages being established between the other carboxylic groups of the benzene tricarboxylic acid - implicated in neither linkage between the 1,2,4,5-benzene tetracarboxylic acid and the polymer nor in the ionic linkages specified above - and $NH_2$ groups of hemoglobin.

18. Procedure according to the Claims 1 to 17, characterized in that at the end of the first step, the polymer containing the Z sites is activated before being made to react with hemoglobin.

19. Procedure according to the Claims 1 to 18, characterized in that the activation consists of converting the OH functions to aldehyde groups by periodate oxidation, for example.

20. Procedure according to the Claims 1 to 19, characterized in that the activation of the Z sites of the polymer and its reaction with hemoglobin are practically simultaneous.

21. Procedure according to the Claims 1 to 20, characterized in that the Z sites of the polymer:

– are activated, for example, using carbonyl diimidazole, when the Z sites do not contain aldehyde groups but when they contain hydroxyl functions,

– or are activated using reagents used in peptide synthesis when the Z sites contain carboxyl groups.

22. Procedure according to the Claims 1 to 21, characterized in that the reaction between the polymer and hemoglobin is carried out for a time shorter than that leading to the formation of more than about 5% of methemoglobin, and preferably not exceeding 10 h, and at a temperature at which the hemoglobin is not denatured, for example, between 3°C and 30°C.

23. Procedure for the preparation according to any one of the Claims 1 to 22 of hemoglobin conjugates in which the Z sites are linked to the polymer by the intermediary of an ester linkage, characterized in that:

– in the first step, the Z sites are made to react in the form of an anhydride, in particular of 1,2,4,5-benzene tetracarboxylic acid dianhydride or of 1,2,4-benzene tricarboxylic acid anhydride, with a polymer containing OH functions, in particular dextran or polyethylene glycol, in a medium in which the polymer is soluble in order to bind the Z sites to the polymer;

– in the second step, the polymer P containing the Z site(s) is made to react with hemoglobin in the oxygenated form in a non-deoxygenated medium under conditions such that the hemoglobin does not undergo denaturation and can pass, after coupling to the polymer, in a reversible manner from the oxygenated form to the deoxygenated form in an aqueous medium at a pH varying between 5 and 9,

in order to form, on the one hand, at least one ionic linkage between at least one of the Z sites borne by the polymer and hemoglobin and, on the other, at least one covalent linkage between the same Z site mentioned aboveborne by the polymer and hemoglobin.

## Patentansprüche

### Patentansprüche für folgende Vertragsstaaten : AT, BE, CH, DE, FR, GB, GR, IT, LI, LU, NL, SE

1. Verfahren zur Herstellung von makromolekularen wasserlöslichen Hämoglobin-Konjugaten, die während der Zeit, in der das makromolekulare Konjugat Sauerstoff-transportierende Funktionen im Plasma sicherstellen muß, nicht oder wenig biologisch abbaubar sind, und die für Sauerstoff eine geringere Affinität besitzen als das freie Hämoglobin, dadurch gekennzeichnet, daß man

– in einer ersten Stufe Stellen Z auf einem Polymeren P fixiert in einem Verhältnis von mindestens eine Stelle Z je Polymerkette, wobei das Polymer P wasserlöslich, nicht-toxisch, vorzugsweise nicht-antigenisch, hämokompatibel ist, eine Molekularmasse von etwa 1000 bis etwa 500 000, vorzugsweise von etwa 1000 bis etwa 100 000, aufweist, eine oder mehrere polare Gruppen, vorzugsweise Hydroxyl-, Carbonsäure- oder Amingruppen, besitzt und die Stellen Z einerseits mindestens eine negative Ladung, getragen von Sulfat- und/oder Phosphat- und/oder Carboxylatgruppen, und zur Erzeugung einer ionischen Bindung mit dem Hämoglobin bestimmt, und andererseits mindestens eine Carbonsäure-, Aldehyd- oder OH-Gruppe, dazu bestimmt, eine kovalente Bindung mit Hämoglobin zu erzeugen, enthalten,

. entweder indem man eine Verbindung Z-Y verwendet, in der Y eine aktive oder mit Hilfe eines Aktivierungsmittels aktivierbare Funktion ist, wie Aldehyd, Carbonsäure, Amin, Hydroxyl oder Halogen, oder

. indem man Stellen Z auf das Polymer P radikalisch aufpfropft;

– daß man dann in einer zweiten Stufe das Polymer P, das die Stelle(n) Z enthält, mit Hämoglobin in oxidierter Form in einem nicht von Sauerstoff befreiten Medium unter solchen Bedingungen umsetzt, daß das Hämoglobin nicht denaturiert wird und nach dem Kuppeln mit dem Polymeren reversibel von der oxidierten in die desoxidierte Form übergehen kann, in wäßrigem Medium vom pH-Wert etwa 5 bis 9,

um einerseits mindestens eine ionische Bindung zwischen mindestens einer der Stellen Z auf dem Polymeren und dem Hämoglobin und andererseits mindestens eine kovalente Bindung zwischen der sleichen genannten Stelle Z auf dem Polymeren und dem Hämoglobin zu bilden,

– wenn die hier oben angegebene Reaktion gegebenenfalls zu Imingruppen führt, diese als Amingruppen stabilisieren kann, beispielsweise durch Reduktion mit Hilfe von $NaBH_4$, $NaCNBH_3$, Dimethylaminoboran oder HCOOH.

2. Verfahren zur Herstellung von makromolekularen Konjugaten nach Anspruch 1, dadurch gekennzeichnet, daß man die Stellen Z auf dem Polymeren P so fixiert, daß die Beziehung zwischen Anzahl und Beschaffenheit der negativen Ladungen, die dazu bestimmt sind, eine ionische Bindung zwischen einer Stelle und dem Hämoglobin (die nicht an der Bindung zwischen einer Stelle und dem Polymeren teilnehmen und nicht an der kovalenten Bindung zwischen einer Stelle und dem Hämoglobin teilnehmen) die folgende ist:

– wenn jede Stelle Z eine einzige anionische Gruppe, bestehend aus einem Sulfat oder einem Phosphat, enthält, gibt es alle zehn Monomer(einheiten) des Polymeren mindestens eine solche Stelle Z,

– wenn es sich um eine Stelle Z handelt, die mindestens zwei anionische Gruppen, bestehend aus Sulfaten und/oder Phosphaten, enthält, gibt es je Polymerkette mindestens eine solche Stelle Z,

– wenn jede Stelle Z von Carboxylaten stammende anionische Ladungen enthält, müssen mindestens zwei Carboxylatgruppen auf einer selben Stelle Z und mindestens eine solche Stelle Z alle fünf Monomer(einheiten) vorhanden sein,

– wenn mindestens eine der Stellen Z mindestens drei von Carboxylaten stammende negative Ladungen enthält, ist mindestens eine solche Stelle je Polymerkette vorhanden.

3. Verfahren zur Herstellung von makromolekularen Konjugaten nach Anspruch 1, dadurch gekennzeichnet, daß man die Stellen Z auf dem Polymeren P so fixiert, daß die Beziehung zwischen Anzahl und Beschaffenheit der zur Erzeugung einer ionischen Bindung zwischen einer Stelle und dem Hämoglobin bestimmten negativen Ladungen (die nicht an der Bindung zwischen einer Stelle und dem Polymeren und nicht an der kovalenten Bindung zwischen einer Stelle und dem Hämoglobin teilnehmen) die folgende ist:

– wenn es sich um eine Stelle Z handelt, die eine einzige anionische Gruppe, bestehend aus einem Sulfat oder einem Phosphat, enthält, ist mindestens eine solche Stelle Z je Polymerkette und höchstens eine Stelle Z alle elf Monomer(einheiten) vorhanden,

– wenn es sich um eine Stelle Z handelt, die eine einzige anionische Gruppe, bestehend aus einem Carboxylat, enthält, die nicht an der Bindung zwischen der Stelle und dem Polymeren und nicht an der kovalenten Bindung zwischen dem Polymeren und dem Hämoglobin teilnimmt, ist mindestens eine solche Stelle Z je Polymerkette vorhanden,

– wenn es sich um eine Stelle Z handelt, die zwei anionische Ladungen enthält, die von zwei Carboxylaten stammen, die nicht an der Bindung zwischen der Stelle und dem Polymeren und nicht an der kovalenten Bindung zwischen dem Polymeren und dem Hämoglobin teilnehmen, ist mindestens eine Stelle Z je Polymerkette und höchstens eine solche Stelle Z alle sechs Monomer(einheiten) vorhanden.

4. Verfahren nach den Ansprüchen 1 bis 3, dadurch gekennzeichnet, daß die kovalenten Bindungen zwischen dem Polymeren P und dem Hämoglobin zwischen mindestens einer Carboxyl-, Aldehyd- oder OH-Gruppe der Stellen Z und mindestens einem Amin des Hämoglobins, gelegen in der allosterischen Stelle des Hämoglobins, wenn dieses in desoxidierter Form vorliegt, vor allem dem Amin von mindestens einem der beiden β-terminalen Valine des Hämoglobins hergestellt werden.

5. Verfahren nach den Ansprüchen 1 bis 4, dadurch gekennzeichnet, daß die Salzbrücken zwischen den inneren $NH_3^+$- und $COO^-$-Gruppen des Hämoglobins intakt sind, wenn das Hämoglobin in desoxidierter Form vorliegt.

6. Verfahren nach den Ansprüchen 1 bis 5, dadurch gekennzeichnet, daß das Polymer P aus den Polysacchariden, vor allem den Hydroxyalkylstärken, deren Alkylgruppe 2 bis 4 Kohlenstoffatome enthält, Inulin, Dextran und seinen Derivaten, vor allem dem aminierten Dextran, Polyvinylalkohol, Polyvinylpyrrolidon, Polymethacrylat und seinen Derivaten, Polypeptiden, Polyalkylenglykolen, deren Alkylengruppe 2 bis 5 Kohlenstoffatome enthält, vor allem Polyethylenglykol und Polypropylenglykol, ausgewählt wird.

7. Verfahren nach den Ansprüchen 1 bis 6, dadurch gekennzeichnet, daß das Polymer P eine mittlere Molekularmasse kleiner oder gleich 70 000 aufweist, wenn es aus Dextran und seinen Derivaten besteht, und

eine Molekularmasse kleiner oder gleich 10 000, wenn es aus den Polyalkylenglykolen, Polyvinylpyrrolidon oder Polymethylacrylat ausgewählt ist.

8. Verfahren nach den Ansprüchen 1 bis 7, dadurch gekennzeichnet, daß die Stelle Z über eine Ester-, Ether-, Amid- oder Amingruppe an das Polymer gebunden ist.

9. Verfahren nach den Ansprüchen 1 bis 8, dadurch gekennzeichnet, daß die Stelle Z die Gruppen $OSO_3H$, $OPO_3H_2$, $O\text{-}CH(COOH)_2$, $\text{-}O\text{-}CH(COOH)\text{-}CH_2\text{-}COOH$,

$$O-CH_2-\left[(\underset{COOH}{CH}-CH_2)\right]_n-CH_2-COOH$$

umfaßt, wobei n 1 bis etwa 4 sein kann, oder von Pyridoxalsulfat, Pyridoxylphosphat, Adenosintriphosphat, Phosphotyrosin, Phosphoserin, Inosithexaphosphat und seinen Derivaten, einer Polycarbonsäure mit 2 bis 10 Kohlenstoffatomen in der Hauptkette, Benzolcarbonsäure mit mindestens drei Carbonsäuregruppen oder 2,3-Diphosphoglycerat stammt.

10. Verfahren nach den Ansprüchen 1 bis 9, dadurch gekennzeichnet, daß die kovalenten Bindungen zwischen dem Polymeren und dem Hämoglobin ausgehend von Carboxylgruppen, die von an das Polymer gebundenen Stellen Z stammen, und $NH_2$-Gruppen des Hämoglobins erzeugt werden.

11. Verfahren nach den Ansprüchen 1 bis 10, dadurch gekennzeichnet, daß die ionischen Bindungen zwischen dem Polymeren P und dem Hämoglobin zwischen den Carboxylatgruppen der Stellen Z und dem Hämoglobin erzeugt werden.

12. Verfahren nach Anspruch 1, dadurch gekennzeichnet, daß das Polymer ausgehend von aminiertem Dextran mit einer Molekularmasse von etwa 40 000 und von $2x10^{-4}$ mol Benzolhexacarbonsäure je g Dextran erhalten wird, wobei

– die ionischen Bindungen mit Hilfe der Carboxylatgruppen des an das Polymeren gebundenen Benzolpentacarboxylats, die nicht an der Bindung zwischen dem Polymeren und der Benzolhexacarbonsäure teilnehmen, hergestellt werden,

– und die kovalenten Bindungen zwischen den anderen Carboxylgruppen der Benzolpentacarbonsäure - die gleichzeitig nicht an der Bindung zwischen Benzolhexacarbonsäure und dem Polymeren und nicht an den hier oben definierten ionischen Bindungen teilnehmen - und $NH_2$-Gruppen des Hämoglobins hergestellt werden.

13. Verfahren nach Anspruch 1, dadurch gekennzeichnet, daß das Polymer ausgehend von aminiertem Dextran mit einer Molekularmasse von etwa 10 000 und von $3{,}5x10^{-4}$ mol Benzolhexacarbonsäure je g Dextran erhalten wird, wobei

– die ionischen Bindungen mit Hilfe der Carboxylatgruppen des auf dem Polymeren fixierten Benzolpentacarboxylats, die nicht an der Bindung zwischen dem Polymeren und der Benzolhexacarbonsäure teilnehmen, erzeugt werden,

– und die kovalenten Bindungen zwischen den anderen Carboxylgruppen der Benzolpentacarbonsäure - die gleichzeitig weder an der Bindung zwischen der Benzolhexacarbonsäure und dem Polymeren noch an den hier oben definierten ionischen Bindungen beteiligt sind - und $NH_2$-Gruppen des Hämoglobins erzeugt werden.

14. Verfahren nach Anspruch 1, dadurch gekennzeichnet, daß das Polymer ausgehend von aminiertem Dextran mit einer Molekularmasse von etwa 10 000 und von $3{,}2x10^{-4}$ mol Benzoltetracarbonsäure je g Dextran erhalten wird, wobei

– die ionischen Bindungen mit Hilfe von Carboxylatgruppen des auf dem Polymeren fixierten Benzoltricarboxylats, die nicht an der Bindung zwischen dem Polymeren und der Benzoltetracarbonsäure beteiligt sind, erzeugt werden,

– und die kovalenten Bindungen zwischen den anderen Carboxylgruppen der Benzoltricarbonsäure - die gleichzeitig weder an der Bindung zwischen Benzoltetracarbonsäure und dem Polymeren noch an den hier oben definierten ionischen Bindungen beteiligt sind - und $NH_2$-Gruppen des Hämoglobins erzeugt werden.

15. Verfahren nach Anspruch 1, dadurch gekennzeichnet, daß das Polymer ausgehend von aminiertem Dextran mit einer Molekularmasse von etwa 10 000 und von $4x10^{-4}$ mol Butantetracarbonsäure je g Polymer erhalten wird, wobei

– die ionischen Bindungen mit Hilfe der Carboxylatgruppen des auf dem Polymeren fixierten Butantricarboxylats, die nicht an der Bindung zwischen dem Polymeren und der Butantetracarbonsäure beteiligt sind, erzeugt werden,

– und die kovalenten Bindungen zwischen den anderen Carboxylgruppen der Butantricarbonsäure - die

gleichzeitig weder an der Bindung zwischen der Butantetracarbonsäure und dem Polymeren noch an den hier oben definierten ionischen Bindungen beteiligt sind - und $NH_2$-Gruppen des Hämoglobins erzeugt werden.

16. Verfahren nach Anspruch 1, dadurch gekennzeichnet, daß das Polymer ausgehend von aminiertem Monomethoxypolyoxyethylen mit einer Molekularmasse von etwa 5000 und von $1,5x10^{-4}$ mol Benzolhexacarbonsäure je g Polymer erhalten wird, wobei

– die ionischen Bindungen mit Hilfe von Carboxylatgruppen des auf dem Polymeren fixierten Benzolpentacarboxylats, die nicht an der Bindung zwischen dem Polymeren und der Benzolhexacarbonsäure beteiligt sind, erzeugt werden,

– und die kovalenten Bindungen zwischen den anderen Carboxylgruppen der Benzolpentacarbonsäure - die gleichzeitig weder an der Bindung zwischen der Benzolhexacarbonsäure und dem Polymeren noch an den hier oben definierten ionischen Bindungen beteiligt sind - und $NH_2$-Gruppen des Hämoglobins erzeugt werden.

17. Verfahren nach Anspruch 1, dadurch gekennzeichnet, daß das Polymer ausgehend von Dextran mit einer Molekularmasse von etwa 10 000 und von $1,15x10^{-3}$ mol Benzol-1,2,4,5-tetracarbonsäure je g Polymer erhalten wird, wobei

– die ionischen Bindungen mit Hilfe von Carboxylatgruppen des auf dem Polymeren fixierten Benzoltricarboxylats, die nicht an der Bindung zwischen dem Polymeren und der Benzol-1,2,4,5-tetracarbonsäure beteiligt sind, erzeugt werden,

– und die kovalenten Bindungen zwischen den anderen Carboxylgruppen der Benzoltricarbonsäure - die gleichzeitig weder an der Bindung zwischen der Benzol-1,2,4,5-tetracarbonsäure und dem Polymeren noch an den hier oben definierten ionischen Bindungen beteiligt sind - und $NH_2$-Gruppen des Hämoglobins erzeugt werden.

18. Verfahren nach den Ansprüchen 1 bis 17, dadurch gekennzeichnet, daß am Ende der ersten Stufe das Polymer mit den Stellen Z vor der Reaktion mit dem Hämoglobin aktiviert wird.

19. Verfahren nach den Ansprüchen 1 bis 18, dadurch gekennzeichnet, daß die Aktivierung darin besteht, daß man die OH-Gruppen in Aldehydgruppen überführt, beispielsweise durch periodische Oxidation.

20. Verfahren nach den Ansprüchen 1 bis 19, dadurch gekennzeichnet, daß die Aktivierung der Stellen Z des Polymeren und seine Reaktion mit dem Hämoglobin praktisch gleichzeitig ablaufen.

21. Verfahren nach den Ansprüchen 1 bis 20, dadurch gekennzeichnet, daß die Stellen Z des Polymeren

– mit beispielsweise Carbonyldiimidazol aktiviert werden, wenn die Stellen Z keine Aldehydgruppen, aber Hydroxylgruppen enthalten,

– oder mit Hilfe von in der Peptidsynthese verwendeten Reaktionspartnern aktiviert werden, wenn die Stellen Z Carboxylgruppen enthalten.

22. Verfahren nach den Ansprüchen 1 bis 21, dadurch gekennzeichnet, daß die Reaktion zwischen dem Polymeren und dem Hämoglobin während einer Zeit, die kürzer ist als die Zeit, die zu höchstens etwa 5% Methhämoglobin führt, vorzugsweise während höchstens 10 h, und bei einer Temperatur, die eine korrekte Konservierung des Hämoglobins ermöglicht, beispielsweise zwischen 3 und 30°C, abläuft.

23. Verfahren zur Herstellung nach einem der Ansprüche 1 bis 22 von Hämoglobinkonjugaten, bei denen die Stellen Z an das Polymer mit Hilfe einer Esterbindung gebunden sind, dadurch gekennzeichnet, daß man

– in einer ersten Stufe die Stellen Z in Anhydridform vor allem Benzol-1,2,4,5-tetracarbonsäure-dianhydrid oder Benzol-1,2,4-tricarbonsäure-anhydrid, mit einem Polymeren, das OH-Gruppen enthält, vor allem Dextran oder Polyethylenglykol, in einem Medium, in welchem das Polymer löslich ist, umsetzt, um die Stellen Z auf dem Polymeren zu fixieren,

– in einer zweiten Stufe das Polymer P mit der Stelle/den Stellen Z mit Hämoglobin in oxidierter Form in einem nicht von Sauerstoff befreiten Medium unter solchen Bedingungen umsetzt, daß das Hämoglobin nicht denaturiert wird und nach dem Kuppeln mit dem Polymeren reversibel von der oxidierten in die desoxidierte Form übergehen kann, in wäßrigem Medium vom pH-Wert 5 bis 9,

um einerseits mindestens eine ionische Bindung zwischen mindestens einer der Stellen Z auf dem Polymeren und dem Hämoglobin und andererseits mindestens eine kovalente Bindung zwischen der gleichen Stelle Z auf dem Polymeren und dem Hämoglobin zu erzeugen.

24. Makromolekulares Konjugat, dadurch gekennzeichnet, daß es durch das Verfahren nach Anspruch 3 erhalten wird.

25. Makromolekulares wasserlösliches Hämoglobin-Konjugat, das während der Zeit, in der das makromolekulare Konjugat Sauerstoff tansportierende Funktionen im Plasma sicherstellen muß, nicht oder wenig biologisch abbaubar ist und das für Sauerstoff eine geringere Affinität besitzt als das freie Hämoglobin, dadurch gekennzeichnet, daß es besteht aus:

– einerseits Hämoglobin, das reversibel aus der desoxidierten Form in die oxidierte Form übergehen kann,

– andererseits einem wasserlöslichen, nichttoxischen, vorzugsweise nicht-antigenischen und hämokompatiblen Polymeren P, mit einer mittleren Molekularmasse von etwa 1000 bis etwa 500 000, vorzugsweise von etwa 1000 bis etwa 100 000, das eine oder mehrere polare Gruppen, vorzugsweise Hydroxyl-, Carboxyl- oder Amingruppen besitzt,

– wobei das Polymer eine oder mehrere Stellen Z aufweist, die mindestens eine negative Ladung getragen von mindestens einer Gruppe, ausgewählt aus den folgenden Gruppen: Sulfat, Phosphat, Carboxylat und bestimmt zur Ausbildung einer ionischen Bindung mit dem Polymeren enthält, und andererseits mindestens eine Carbonsäure-, Aldehyd- oder OH-Gruppe enthält, die zur Ausbildung einer kovalenten Bindung mit dem Hämoglobin bestimmt ist, wobei

– das Polymer P an das Hämoglobin gebunden ist,

. einerseits mit Hilfe mindestens einer ionischen Bindung, die zwischen mindestens einer der negativen Ladungen der Stellen Z auf dem Polymeren P und dem Hämoglobin ausgebildet ist, und

. andererseits mit Hilfe mindestens einer kovalenten Bindung, die zwischen mindestens einer der Carboxyl-, Aldehyd- oder OH-Gruppen der genannten Stellen Z auf dem Polymeren P und dem Hämoglobin ausgebildet ist,

– die Anzahl der kovalenten Bindungen zwischen dem Polymeren und dem Hämoglobin so ist, daß das makromolekulare Konjugat eine mittleren Molekularmasse von etwa 70 000 bis etwa 1 000 000, vorzugsweise von etwa 70 000 bis etwa 500 000, aufweist und die Beziehung zwischen Anzahl und Beschaffenheit der negativen Ladungen, der Anzahl der Stellen und der Anzahl der Monomeren folgendermaßen lautet:

– handelt es sich um eine Stelle Z, die eine einzige anionische Gruppe, bestehend aus einem Sulfat oder einem Phosphat, enthält, so gibt es mindestens eine solche Stelle Z je Polymerkette und höchstens eine Stelle alle elf Monomer(einheiten),

– handelt es sich um eine Stelle Z, die eine einzige anionische Gruppe enthält, bestehend aus einem Carboxylat, das nicht an der Bindung zwischen der Stelle und dem Polymeren und nicht an der kovalenten Bindung zwischen dem Polymeren und dem Hämoglobin teilnimmt, so gibt es mindestens eine solche Stelle Z je Polymerkette,

– handelt es sich um eine Stelle Z, die zwei anionische Ladungen enthält, die von zwei Carboxylaten stammen, die nicht an der Bindung zwischen der Stelle und dem Polymeren und nicht an der kovalenten Bindung zwischen dem Polymeren und dem Hämoglobin teilnehmen, so gibt es mindestens eine solche Stelle Z je Polymerkette und höchstens eine Stelle Z alle sechs Monomer(einheiten).

26. Makromolekulares Konjugat nach Anspruch 25, dadurch gekennzeichnet, daß die kovalenten Bindungen zwischen dem Polymeren P und dem Hämoglobin zwischen mindestens einer Carboxyl-, Aldehyd- oder OH-Gruppe der Stellen Z und mindestens einem Amin des Hämoglobins, gelegen in der allosterischen Stelle des Hämoglobins, vor allem dem Amin von mindestens einem der beiden β-terminalen Valine des Hämoglobins ausgebildet sind.

27. Makromolekulares Konjugat nach den Ansprüchen 25 und 26, dadurch gekennzeichnet, daß die Salzbrücken zwischen den inneren $NH_3^+$- und $COO^-$-Gruppen des Hämoglobins intakt sind, wenn das Hämoglobin in desoxidierter Form vorliegt.

28. Makromolekulares Konjugat nach den Ansprüchen 26 und 27, dadurch gekennzeichnet, daß das Polymer P aus den Polysacchariden, vor allem den Hydroxyalkylstärken, deren Alkylgruppe 2 bis 4 Kohlenstoffatome enthält, Inulin, Dextran und seinen Derivaten, vor allem dem aminierten Dextran, Polyvinylalkohol, Polyvinylpyrrolidon, Polymethacrylat und seinen Derivaten, Polypeptiden, Polyalkylenglykolen, deren Alkylengruppe 2 bis 5 Kohlenstoffatome enthält, vor allem Polyethylenglykol und Polypropylenglykol, ausgewählt wird.

29. Makromolekulares Konjugat nach den Ansprüchen 25 bis 28, dadurch gekennzeichnet, daß das Polymer P eine mittlere Molekularmasse kleiner oder gleich 70 000 aufweist, wenn es aus Dextran und seinen Derivaten besteht, und eine Molekularmasse kleiner oder gleich 10 000, wenn es aus den Polyalkylenalykolen, Polyvinylpyrrolidon oder Polymethylacrylat ausgewählt ist.

30. Makromolekulares Konjugat nach den Ansprüchen 25 bis 29, dadurch gekennzeichnet, daß die Stelle Z über eine Ester-, Ether-, Amid- oder Amingruppe an das Polymer gebunden ist.

31. Makromolekulares Konjugat nach den Ansprüchen 25 bis 30, dadurch gekennzeichnet, daß die Stelle Z Gruppen $OSO_3H$, $OPO_3H_2$, $O-CH(COOH)_2$, $-O-CH-COOH)-CH_2-COOH$,

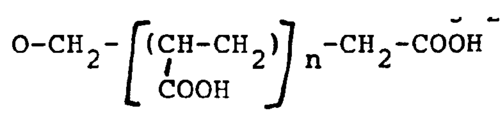

umfaßt, wobei n 1 bis etwa 4 sein kann, oder von Pyridoxalsulfat, Pyridoxylphosphat, Adenosintriphosphat, Phosphotyrosin, Phosphoserin, Inosithexaphosphat und seinen Derivaten, einer Polycarbonsäure mit 2 bis 10 Kohlenstoffatomen in der Hauptkette, Benzolcarbonsäure mit mindestens drei Carbonsäuregruppen oder 2,3-Diphosphoglycerat stammt.

32. Makromolekulares Konjugat nach den Ansprüchen 25 bis 31, dadurch gekennzeichnet, daß die kovalenten Bindungen zwischen dem Polymeren und dem Hämoglobin ausgehend von Carboxylgruppen, die von an das Polymer gebundenen Stellen Z stammen, und $NH_2$-Gruppen des Hämoglobins erzeugt worden sind.

33. Makromolekulares Konjugat nach den Ansprüchen 25 bis 32, dadurch gekennzeichnet, daß die ionischen Bindungen zwischen dem Polymeren P und dem Hämoglobin zwischen den Carboxylatgruppen der Stellen Z und dem Hämoglobin erzeugt werden.

34. Makromolekulares Konjugat nach dem Anspruch 25, bei dem das Polymer Dextran mit einer Molekularmasse von etwa 10 000 ist, die ionischen Bindungen mit Hilfe der Carboxylatgruppe des auf dem Polymeren fixierten Benzoldicarboxylats, die nicht an der Bindung zwischen dem Benzol-1,2,4-tricarbonsäureanhydrid und dem Polymeren teilnimmt, ausgebildet sind (und)

– die kovalenten Bindungen zwischen der anderen Carboxylgruppe des Benzoldicarboxylats - die gleichzeitig weder an der Bindung zwischen dem Benzol-1,2,4-tricarbonsäureanhydrid und dem Polymeren noch an der hier oben definierten ionischen Bindung beteiligt ist - und $NH_2$-Gruppen des Hämoglobins ausgebildet sind, wobei alle zehn Monomer(einheiten) eine Benzoldicarboxylatgruppe vorhanden ist.

**Patentansprüche für folgenden Vertragsstaat : ES**

1. Verfahren zur Herstellung von makromolekularen wasserlöslichen Hämoglobin-Konjugaten, die während der Zeit, in der das makromolekulare Konjugat Sauerstoff-transportierende Funktionen im Plasma sicherstellen muß, nicht oder wenig biologisch abbaubar sind, und die für Sauerstoff eine geringere Affinität besitzen als das freie Hämoglobin, dadurch gekennzeichnet, daß man

– in einer ersten Stufe Stellen Z auf einem Polymeren P fixiert in einem Verhältnis von mindestens eine Stelle Z je Polymerkette, wobei das Polymer P wasserlöslich, nicht-toxisch, vorzugsweise nicht-antigenisch, hämokompatibel ist, eine Molekularmasse von etwa 1000 bis etwa 500 000, vorzuasweise von etwa 1000 bis etwa 100 000, aufweist, eine oder mehrere polare Gruppen, vorzugsweise Hydroxyl-, Carbonsäure- oder Amingruppen, besitzt und die Stellen Z einerseits mindestens eine negative Ladung, getragen von Sulfat- und/oder Phosphat- und/oder Carboxylataruppen, und zur Erzeugung einer ionischen Bindung mit dem Hämoglobin bestimmt, und andererseits mindestens eine Carbonsäure-, Aldehyd- oder OH-Gruppe, dazu bestimmt, eine kovalente Bindung mit Hämoglobin zu erzeugen, enthalten,

. entweder indem man eine Verbindung Z-Y verwendet, in der Y eine aktive oder mit Hilfe eines Aktivierungsmittels aktivierbare Funktion ist, wie Aldehyd, Carbonsäure, Amin, Hydroxyl oder Halogen, oder

. indem man Stellen Z auf das Polymer P radikalisch aufpfropft;

– daß man dann in einer zweiten Stufe das Polymer P, das die Stelle(n) Z enthält, mit Hämoglobin in oxidierter Form in einem nicht von Sauerstoff befreiten Medium unter solchen Bedingungen umsetzt, daß das Hämoglobin nicht denaturiert wird und nach dem Kuppeln mit dem Polymeren reversibel von der oxidierten in die desoxidierte Form übergehen kann, in wäßrigem Medium vom pH-Wert etwa 5 bis 9,

um einerseits mindestens eine ionische Bindung zwischen mindestens einer der Stellen Z auf dem Polymeren und dem Hämoglobin und andererseits mindestens eine kovalente Bindung zwischen der gleichen genannten Stelle Z auf dem Polymeren und dem Hämoglobin zu bilden,

– wenn die hier oben angegebene Reaktion gegebenenfalls zu Imingruppen führt, diese als Amingruppen stabilisieren kann, beispielsweise durch Reduktion mit Hilfe von $NaBH_4$, $NaCNBH_3$, Dimethylaminoboran oder HCOOH.

2. Verfahren zur Herstellung von makromolekularen Konjugaten nach Anspruch 1, dadurch gekennzeichnet, daß man die Stellen Z auf dem Polymeren P so fixiert, daß die Beziehung zwischen Anzahl und Beschaffenheit der negativen Ladungen, die dazu bestimmt sind, eine ionische Bindung zwischen einer Stelle und dem Hämoglobin (die nicht an der Bindung zwischen einer Stelle und dem Polymeren teilnehmen und nicht an der kovalenten Bindung zwischen einer Stelle und dem Hämoglobin teilnehmen) die folgende ist:

– wenn jede Stelle Z eine einzige anionische Gruppe, bestehend aus einem Sulfat oder einem Phosphat, enthält, gibt es alle zehn Monomer(einheiten) des Polymeren mindestens eine solche Stelle Z,

– wenn es sich um eine Stelle Z handelt, die mindestens zwei anionische Gruppen, bestehend aus Sulfaten und/oder Phosphaten, enthält, gibt es je Polymerkette mindestens eine solche Stelle Z,

– wenn jede Stelle Z von Carboxylaten stammende anionische Ladungen enthält, müssen mindestens zwei Carboxylatgruppen auf einer selben Stelle Z und mindestens eine solche Stelle Z alle fünf Monomer(einheiten) vorhanden sein,

– wenn mindestens eine der Stellen Z mindestens drei von Carboxylaten stammende negative Ladungen enthält, ist mindestens eine solche Stelle je Polymerkette vorhanden.

3. Verfahren zur Herstellung von makromolekularen Konjugaten nach Anspruch 1, dadurch gekennzeichnet, daß man die Stellen Z auf dem Polymeren P so fixiert, daß die Beziehung zwischen Anzahl und Beschaffenheit der zur Erzeugung einer ionischen Bindung zwischen einer Stelle und dem Hämoglobin bestimmten negativen Ladungen (die nicht an der Bindung zwischen einer Stelle und dem Polymeren und nicht an der kovalenten Bindung zwischen einer Stelle und dem Hämoglobin teilnehmen) die folgende ist:

– wenn es sich um eine Stelle Z handelt, die eine einzige anionische Gruppe, bestehend aus einem Sulfat oder einem Phosphat, enthält, ist mindestens eine solche Stelle Z je Polymerkette und höchstens eine Stelle Z alle elf Monomer(einheiten) vorhanden,

– wenn es sich um eine Stelle Z handelt, die eine einzige anionische Gruppe, bestehend aus einem Carboxylat, enthält, die nicht an der Bindung zwischen der Stelle und dem Polymeren und nicht an der kovalenten Bindung zwischen dem Polymeren und dem Hämoglobin teilnimmt, ist mindestens eine solche Stelle Z je Polymerkette vorhanden,

– wenn es sich um eine Stelle Z handelt, die zwei anionische Ladungen enthält, die von zwei Carboxylaten stammen, die nicht an der Bindung zwischen der Stelle und dem Polymeren und nicht an der kovalenten Bindung zwischen dem Polymeren und dem Hämoglobin teilnehmen, ist mindestens eine Stelle Z je Polymerkette und höchstens eine solche Stelle Z alle sechs Monomer(einheiten) vorhanden.

4. Verfahren nach den Ansprüchen 1 bis 3, dadurch gekennzeichnet, daß die kovalenten Bindungen zwischen dem Polymeren P und dem Hämoglobin zwischen mindestens einer Carboxyl-, Aldehyd- oder OH-Gruppe der Stellen Z und mindestens einem Amin des Hämoglobins, gelegen in der allosterischen Stelle des Hämoglobins, wenn dieses in desoxidierter Form vorliegt, vor allem dem Amin von mindestens einem der beiden β-terminalen Valine des Hämoglobins hergestellt werden.

5. Verfahren nach den Ansprüchen 1 bis 4, dadurch gekennzeichnet, daß die Salzbrücken zwischen den inneren $NH_3^+$- und $COO^-$ -Gruppen des Hämoglobins intakt sind, wenn das Hämoglobin in desoxidierter Form vorliegt.

6. Verfahren nach den Ansprüchen 1 bis 5, dadurch gekennzeichnet, daß das Polymer P aus den Polysacchariden, vor allem den Hydroxyalkylstärken, deren Alkylgruppe 2 bis 4 Kohlenstoffatome enthält, Inulin, Dextran und seinen Derivaten, vor allem dem aminierten Dextran, Polyvinylalkohol, Polyvinylpyrrolidon, Polymethacrylat und seinen Derivaten, Polypeptiden, Polyalkylenglykolen, deren Alkylengruppe 2 bis 5 Kohlenstoffatome enthält, vor allem Polyethylenglykol und Polypropylenglykol, ausgewählt wird.

7. Verfahren nach den Ansprüchen 1 bis 6, dadurch gekennzeichnet, daß das Polymer P eine mittlere Molekularmasse kleiner oder gleich 70 000 aufweist, wenn es aus Dextran und seinen Derivaten besteht, und eine Molekularmasse kleiner oder gleich 10 000, wenn es aus den Polyalkylenglykolen, Polyvinylpyrrolidon oder Polymethylacrylat ausgewählt ist.

8. Verfahren nach den Ansprüchen 1 bis 7, dadurch gekennzeichnet, daß die Stelle Z über eine Ester-, Ether-, Amid- oder Amingruppe an das Polymer gebunden ist.

9. Verfahren nach den Ansprüchen 1 bis 8, dadurch gekennzeichnet, daß die Stelle Z die Gruppen $OSO_3H$, $OPO_3H_2$, $O-CH(COOH)_2$, $-O-CH(COOH)-CH_2-COOH$,

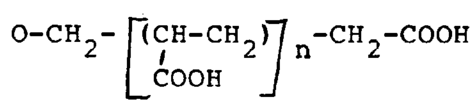

umfaßt, wobei n 1 bis etwa 4 sein kann, oder von Pyridoxalsulfat, Pyridoxylphosphat, Adenosintriphosphat, Phosphotyrosin, Phosphoserin, Inosithexaphosphat und seinen Derivaten, einer Polycarbonsäure mit 2 bis 10 Kohlenstoffatomen in der Hauptkette, Benzolcarbonsäure mit mindestens drei Carbonsäuregruppen oder 2,3-Diphosphoglycerat stammt.

10. Verfahren nach den Ansprüchen 1 bis 9, dadurch gekennzeichnet, daß die kovalenten Bindungen zwischen dem Polymeren und dem Hämoglobin ausgehend von Carboxylgruppen, die von an das Polvmer gebundenen Stellen Z stammen, und $NH_2$-Gruppen des Hämoglobins erzeugt werden.

11. Verfahren nach den Ansprüchen 1 bis 10, dadurch gekennzeichnet, daß die ionischen Bindungen zwischen dem Polymeren P und dem Hämoglobin zwischen den Carboxylatgruppen der Stellen Z und dem Hämoglobin erzeugt werden.

12. Verfahren nach Anspruch 1, dadurch gekennzeichnet, daß das Polymer ausgehend von aminiertem Dextran mit einer Molekularmasse von etwa 40 000 und von $2x10^{-4}$ mol Benzolhexacarbonsäure je g Dextran erhalten wird, wobei

– die ionischen Bindungen mit Hilfe der Carboxylatgruppen des an das Polymeren gebundenen Benzolpentacarboxylats, die nicht an der Bindung zwischen dem Polymeren und der Benzolhexacarbonsäure teilnehmen, hergestellt werden,

– und die kovalenten Bindungen zwischen den anderen Carboxylgruppen der Benzolpentacarbonsäure - die gleichzeitig nicht an der Bindung zwischen Benzolhexacarbonsäure und dem Polymeren und nicht an den hier oben definierten ionischen Bindungen teilnehmen - und $NH_2$-Gruppen des Hämoglobins hergestellt werden.

13. Verfahren nach Anspruch 1, dadurch gekennzeichnet, daß das Polymer ausgehend von aminiertem Dextran mit einer Molekularmasse von etwa 10 000 und von $3{,}5 \times 10^{-4}$ mol Benzolhexacarbonsäure je g Dextran erhalten wird, wobei

– die ionischen Bindungen mit Hilfe der Carboxylatgruppen des auf dem Polymeren fixierten Benzolpentacarboxylats, die nicht an der Bindung zwischen dem Polymeren und der Benzolhexacarbonsäure teilnehmen, erzeugt werden,

– und die kovalenten Bindungen zwischen den anderen Carboxylgruppen der Benzolpentacarbonsäure - die gleichzeitig weder an der Bindung zwischen der Benzolhexacarbonsäure und dem Polymeren noch an den hier oben definierten ionischen Bindungen beteiligt sind - und $NH_2$-Gruppen des Hämoglobins erzeugt werden.

14. Verfahren nach Anspruch 1, dadurch gekennzeichnet, daß das Polymer ausgehend von aminiertem Dextran mit einer Molekularmasse von etwa 10 000 und von $3{,}2 \times 10^{-4}$ mol Benzoltetracarbonsäure je g Dextran erhalten wird, wobei

– die ionischen Bindungen mit Hilfe von Carboxylatgruppen des auf dem Polymeren fixierten Benzoltricarboxylats, die nicht an der Bindung zwischen dem Polymeren und der Benzoltetracarbonsäure beteiligt sind, erzeugt werden,

– und die kovalenten Bindungen zwischen den anderen Carboxylgruppen der Benzoltricarbonsäure - die gleichzeitig weder an der Bindung zwischen Benzoltetracarbonsäure und dem Polymeren noch an den hier oben definierten ionischen Bindungen beteiligt sind - und $NH_2$-Gruppen des Hämoglobins erzeugt werden.

15. Verfahren nach Anspruch 1, dadurch gekennzeichnet, daß das Polymer ausgehend von aminiertem Dextran mit einer Molekularmasse von etwa 10 000 und von $4 \times 10^{-4}$ mol Butantetracarbonsäure je g Polymer erhalten wird, wobei

– die ionischen Bindungen mit Hilfe der Carboxylatgruppen des auf dem Polymeren fixierten Butantricarboxylats, die nicht an der Bindung zwischen dem Polymeren und der Butantetracarbonsäure beteiligt sind, erzeugt werden,

– und die kovalenten Bindungen zwischen den anderen Carboxylgruppen der Butantricarbonsäure - die gleichzeitig weder an der Bindung zwischen der Butantetracarbonsäure und dem Polymeren noch an den hier oben definierten ionischen Bindungen beteiligt sind - und $NH_2$-Gruppen des Hämoglobins erzeugt werden.

16. Verfahren nach Anspruch 1, dadurch gekennzeichnet, daß das Polymer ausgehend von aminiertem Monomethoxypolyoxyethylen mit einer Molekularmasse von etwa 5000 und von $1{,}5 \times 10^{-4}$ mol Benzolhexacarbonsäure je g Polymer erhalten wird, wobei

– die ionischen Bindungen mit Hilfe von Carboxylatgruppen des auf dem Polymeren fixierten Benzolpentacarboxylats, die nicht an der Bindung zwischen dem Polymeren und der Benzolhexacarbonsäure beteiligt sind, erzeugt werden,

– und die kovalenten Bindungen zwischen den anderen Carboxylgruppen der Benzolpentacarbonsäure - die gleichzeitig weder an der Bindung zwischen der Benzolhexacarbonsäure und dem Polymeren noch an den hier oben definierten ionischen Bindungen beteiligt sind - und $NH_2$-Gruppen des Hämoglobins erzeugt werden.

17. Verfahren nach Anspruch 1, dadurch gekennzeichnet, daß das Polymer ausgehend von Dextran mit einer Molekularmasse von etwa 10 000 und von $1{,}15 \times 10^{-3}$ mol Benzol-1,2,4,5-tetracarbonsäure je g Polymer erhalten wird, wobei

– die ionischen Bindungen mit Hilfe von Carboxylatgruppen des auf dem Polymeren fixierten Benzoltricarboxylats, die nicht an der Bindung zwischen dem Polymeren und der Benzol-1,2,4,5-tetracarbonsäure beteiligt sind, erzeugt werden,

– und die kovalenten Bindungen zwischen den anderen Carboxylgruppen der Benzoltricarbonsäure - die gleichzeitig weder an der Bindung zwischen der Benzol-1,2,4,5-tetracarbonsäure und dem Polymeren noch an den hier oben definierten ionischen Bindunaen beteiligt sind - und $NH_2$-Gruppen des Hämoglobins erzeugt werden.

18. Verfahren nach den Ansprüchen 1 bis 17, dadurch gekennzeichnet, daß am Ende der ersten Stufe das Polymer mit den Stellen Z vor der Reaktion mit dem Hämoglobin aktiviert wird.

19. Verfahren nach den Ansprüchen 1 bis 18, dadurch gekennzeichnet, daß die Aktivierung darin besteht, daß man die OH-Gruppen in Aldehydgruppen überführt, beispielsweise durch periodische Oxidation.

20. Verfahren nach den Ansprüchen 1 bis 19, dadurch gekennzeichnet, daß die Aktivierung der Stellen Z des Polymeren und seine Reaktion mit dem Hämoglobin praktisch gleichzeitig ablaufen.

21. Verfahren nach den Ansprüchen 1 bis 20, dadurch gekennzeichnet, daß die Stellen Z des Polymeren

– mit beispielsweise Carbonyldiimidazol aktiviert werden, wenn die Stellen Z keine Aldehydgruppen, aber Hydroxylgruppen enthalten,

– oder mit Hilfe von in der Peptidsynthese verwendeten Reaktionspartnern aktiviert werden, wenn die Stellen Z Carboxylgruppen enthalten.

22. Verfahren nach den Ansprüchen 1 bis 21, dadurch gekennzeichnet, daß die Reaktion zwischen dem Polymeren und dem Hämoglobin während einer Zeit, die kürzer ist als die Zeit, die zu höchstens etwa 5% Methhämoglobin führt, vorzugsweise während höchstens 10 h, und bei einer Temperatur, die eine korrekte Konservierung des Hämoglobins ermöglicht, beispielsweise zwischen 3 und 30°C, abläuft.

23. Verfahren zur Herstellung nach einem der Ansprüche 1 bis 22 von Hämoglobinkonjugaten, bei denen die Stellen Z an das Polymer mit Hilfe einer Esterbindung gebunden sind, dadurch gekennzeichnet, daß man

– in einer ersten Stufe die Stellen Z in Anhydridform vor allem Benzol-1,2,4,5-tetracarbonsäure-dianhydrid oder Benzol-1,2,4-tricarbonsäure-anhydrid, mit einem Polymeren, das OH-Gruppen enthält, vor allem Dextran oder Polyethylenglykol, in einem Medium, in welchem das Polymer löslich ist, umsetzt, um die Stellen Z auf dem Polymeren zu fixieren,

– in einer zweiten Stufe das Polymer P mit der Stelle/den Stellen Z mit Hämoglobin in oxidierter Form in einem nicht von Sauerstoff befreiten Medium unter solchen Bedingungen umsetzt, daß das Hämoglobin nicht denaturiert wird und nach dem Kuppeln mit dem Polymeren reversibel von der oxidierten in die desoxidierte Form übergehen kann, in wäßrigem Medium vom pH-Wert 5 bis 9,

um einerseits mindestens eine ionische Bindung zwischen mindestens einer der Stellen Z auf dem Polymeren und dem Hämoglobin und andererseits mindestens eine kovalente Bindung zwischen der gleichen Stelle Z auf dem Polymeren und dem Hämoglobin zu erzeugen.

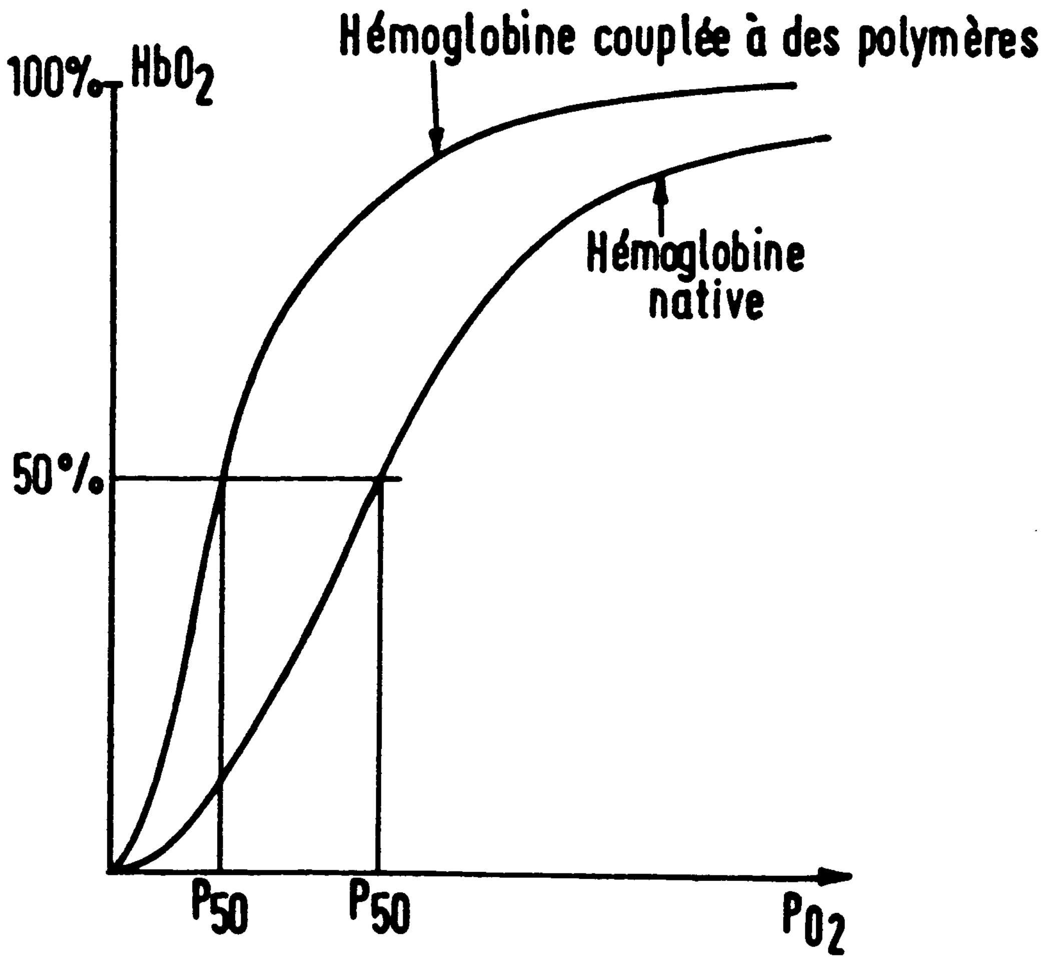
Hémoglobine couplée à des polymères
100%
HbO2
Hémoglobine native
50%
P50
P50
PO2

FIG.1

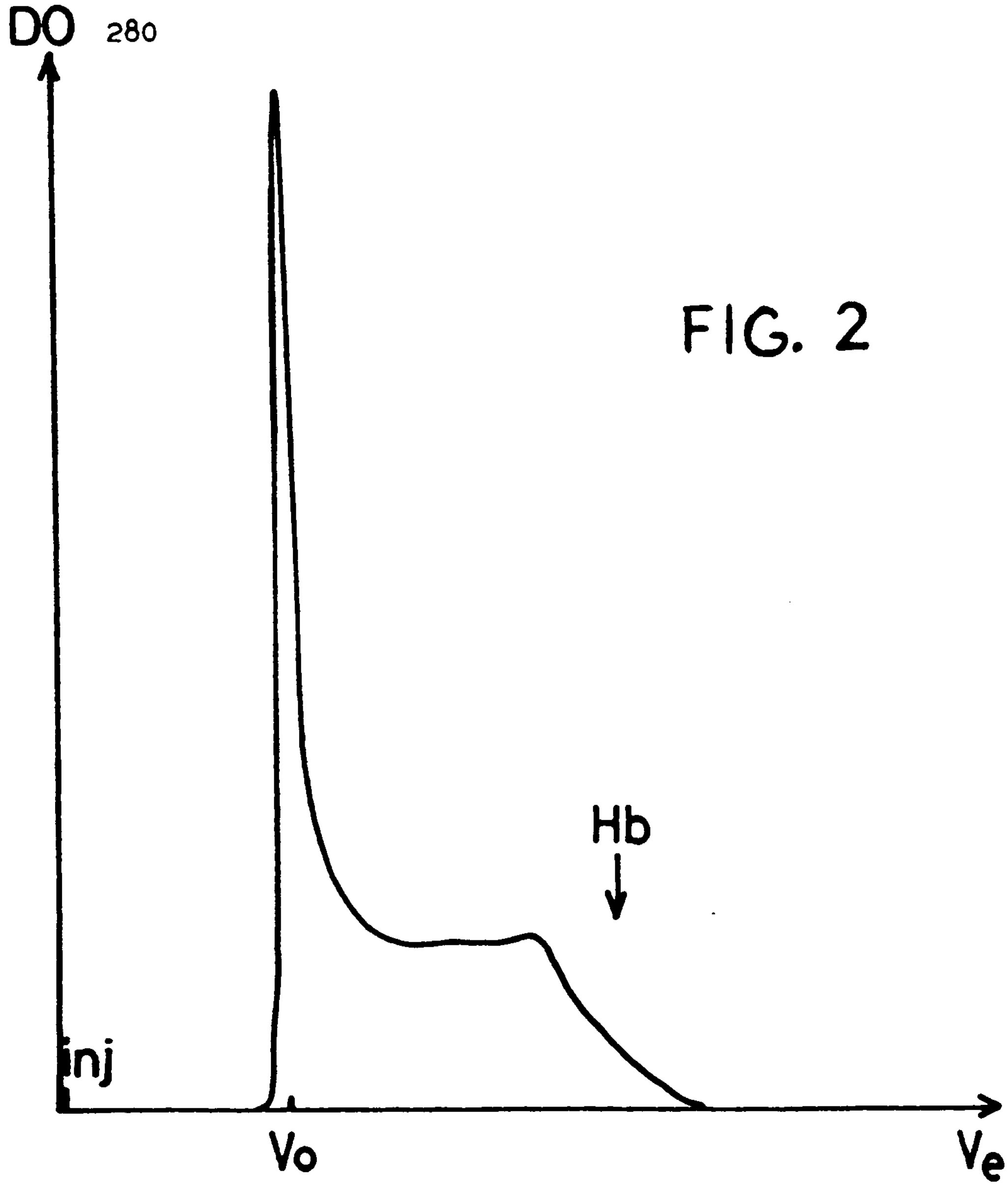


FIG. 2

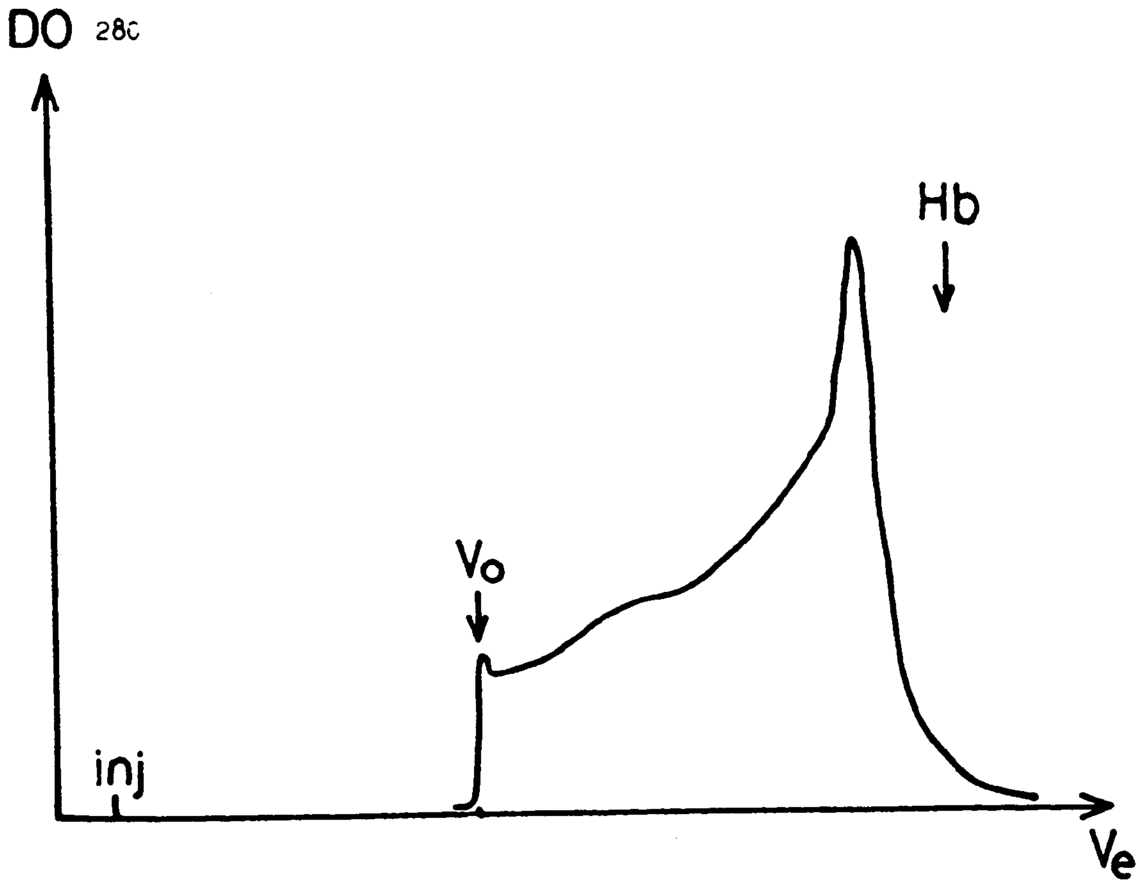


FIG. 3

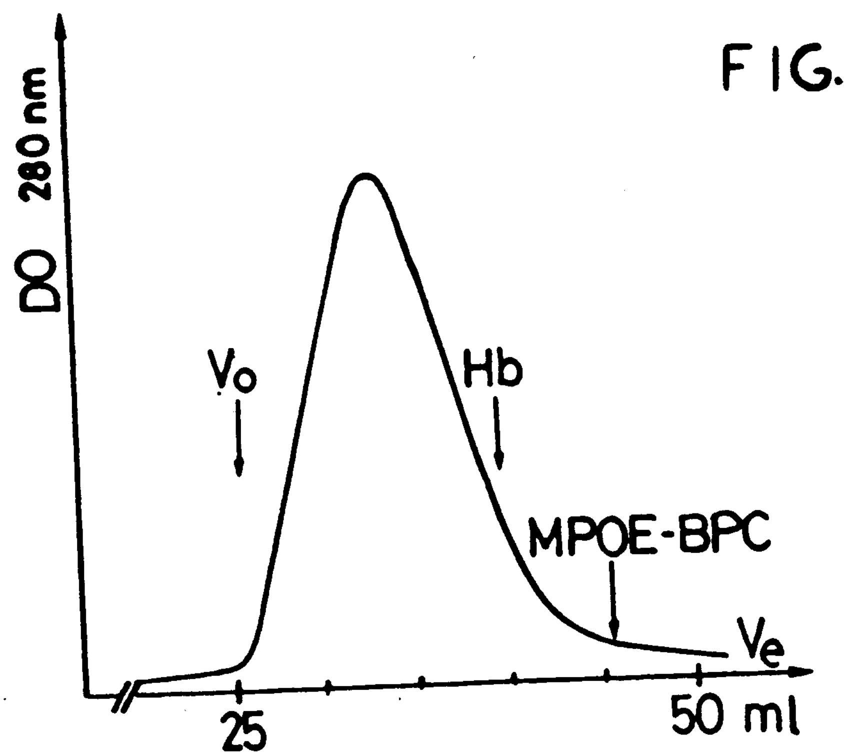


FIG. 4